(19) Europäisches Patentamt
European Patent Office
Office européen des brevets

(11) EP 0 781 283 B1

(12) **EUROPEAN PATENT SPECIFICATION**

(45) Date of publication and mention of the grant of the patent: **06.06.2001 Bulletin 2001/23**

(21) Application number: **95930729.9**

(22) Date of filing: **11.09.1995**

(51) Int Cl.[7]: **C07D 487/04**, A61K 31/50 // (C07D487/04, 249:00, 237:00)

(86) International application number: **PCT/JP95/01797**

(87) International publication number: **WO 96/08496 (21.03.1996 Gazette 1996/13)**

# (54) TRIAZOLOPYRIDAZINES PROCESS AND INTERMEDIATES FOR THEIR PREPARATION AND THEIR USE AS MEDICAMENTS

TRIAZOLOPYRIDAZINE, VERFAHREN UND ZWISCHENPRODUKTE ZU IHRER HERSTELLUNG UND IHRE VERWENDUNG ALS ARZNEIMITTEL

ELABORATION DE TRIAZOLOPYRIDAZINES ET DES INTERMEDIAIRES DESTINES A LEUR PREPARATION, ET LEUR UTILISATION COMME MEDICAMENTS

(84) Designated Contracting States: **AT BE CH DE DK ES FR GB GR IE IT LI LU NL PT SE**

(30) Priority: **16.09.1994 US 306423**
**22.09.1994 JP 22748194**

(43) Date of publication of application: **02.07.1997 Bulletin 1997/27**

(73) Proprietor: **Takeda Chemical Industries, Ltd. Osaka-shi, Osaka 541-0045 (JP)**

(72) Inventors:
- **KAWANO, Yasuhiko Osaka 565 (JP)**
- **MIYAKE, Akio Osaka 573 (JP)**
- **ASHIDA, Yasuko Osaka 569 (JP)**

(74) Representative: **von Kreisler, Alek, Dipl.-Chem. et al Patentanwälte, von Kreisler-Selting-Werner, Bahnhofsvorplatz 1 (Deichmannhaus) 50667 Köln (DE)**

(56) References cited:
**EP-A- 0 548 923**
**EP-A- 0 562 439**
**EP-A- 0 648 491**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European Patent Convention).

EP 0 781 283 B1

## Description

Technical Field

The present invention relates to a novel triazolopyridazine derivative exhibiting excellent anti-allergic, anti-inflammatory, anti-PAF (platelet activating factor), eosinophil chemotaxis inhibiting and other actions, its production and use.

Background Art

Many compounds having a triazolopyridazine skeleton are currently synthesized as drugs for a variety of diseases. For example, USP3,915,968 discloses a compound of the formula:

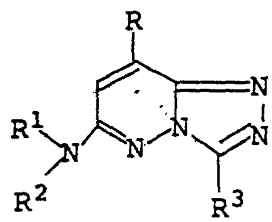

wherein R and $R^3$ are independently a hydrogen atom or a lower alkyl, at least one of R and $R^3$ is a lower alkyl; $R^1$ and $R^2$ form a nitrogen-containing heterocyclic group together with the adjacent nitrogen atom, for example, pyrrolidine, piperidine, piperazine, and morpholine, or a salt thereof. USP4,136,182 discloses a compound of the formula:

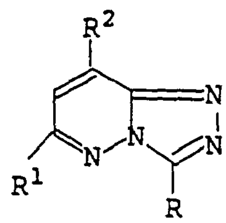

wherein R is a hydrogen atom, a phenyl or a lower alkylcarbonylamino; $R^1$ is morpholino or piperidino; $R^2$ is a hydrogen atom or a lower alkyl, at least one of R and $R^2$ is a group excluding a hydrogen atom; when R is a phenyl, $R^1$ is morpholino and $R^2$ is a lower alkyl, or a salt thereof. EP248413 discloses a compound of the formula:

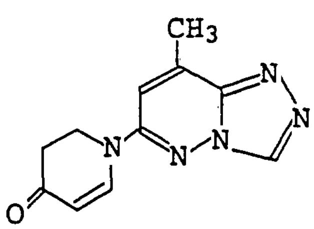

or a salt thereof, as useful as bronchodilators for mitigating bronchial spasms.

Also, EP562439 discloses a compound of the formula:

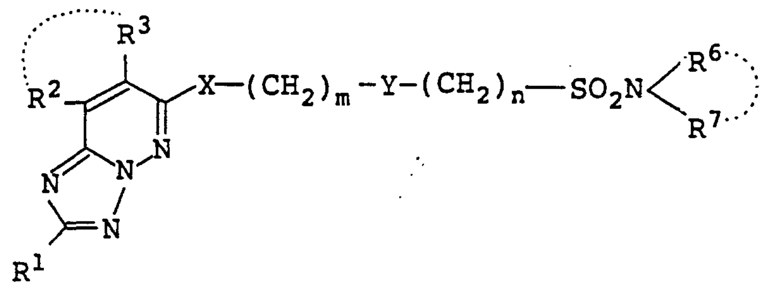

wherein

$R^1$ is a hydrogen atom, an optionally substituted lower alkyl group or a halogen atom;
$R^2$ and $R^3$ are independently a hydrogen atom or an optionally substituted lower alkyl group, or may form a 5-to 7-membered ring together with the adjacent -C=C-;
X is an oxygen atom or $S(O)_p$, wherein p is an integer of 0 to 2;
Y is a group of the formula:

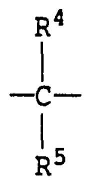

wherein $R^4$ and $R^5$ are independently a hydrogen atom or an optionally substituted lower alkyl group or a divalent group derived from a 3- to 7-membered homocycle or heterocycle which is optionally substituted;
$R^6$ and $R^7$ are independently a hydrogen atom, an optionally substituted lower alkyl group, an optionally substituted cycloalkyl group, or an optionally substituted aryl group, or may form a nitrogen-containing heterocyclic group which is optionally substituted, together with the adjacent nitrogen atom;
m is an integer of 0 to 4, and n is an integer of 0 to 4,
or a salt thereof; and, as an example of synthetic product, a compound of the formula:

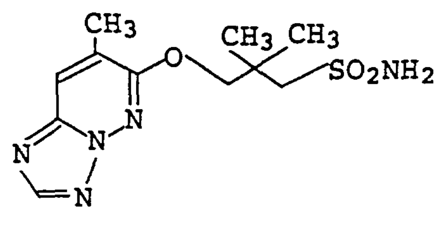

which are described as exhibiting anti-asthmatic, anti-PAF, anti-inflammatory and anti-allergic actions.

EP-A-0648491 describes a composition for inhibiting an eosinophil chemotaxis which comprises a compound of the formula:

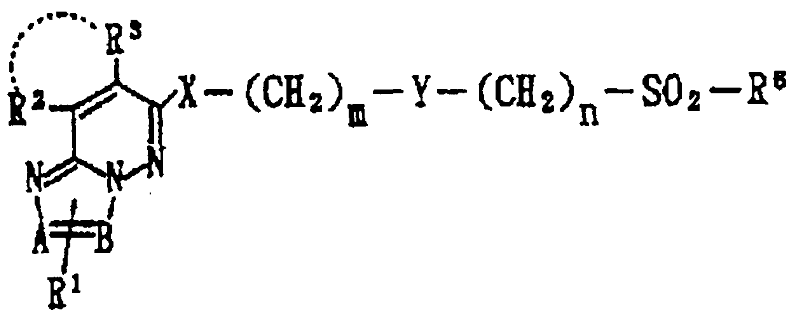

wherein

$R^1$ represents a hydrogen atom, an optionally substituted lower alkyl group or a halogen atom; either A or B represents a nitrogen atom with the other representing a methine group, or both of them represents a methine group; $R^2$ and $R^3$ independently represent a hydrogen atom or an optionally substituted lower alkyl group, or $R^2$ and $R^3$ taken together with the adjacent -C=C-, may form a 5- to 7-membered ring;
X represents a methylene group, an oxygen atom or S(O)p wherein p represents an integer of 0 to 2;
Y represents (i) a group of the formula

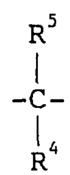

wherein

$R^4$ and $R^5$ independently represent a hydrogen atom or an optionally substituted lower alkyl group or (ii) a divalent group derived from an optionally substituted
3- to 7-membered homocyclic or heterocyclic ring;
$R^6$ represents a primary to tertiary amino group;
m and n independently represent an integer of 0 to 4,
or a salt thereof.

Said composition is useful for treating a disease related to eosinophil chemotaxis such as allergic rhinitis and/or atopic dermatitis and so on.

Disclosure of Invention

There is demand for the development of a novel compound more satisfactory than conventional anti-asthmatics, anti-allergic agents etc., in terms of action efficacy, sustained action, safety etc.
The present invention relates to:

(1) compounds of the formula I;

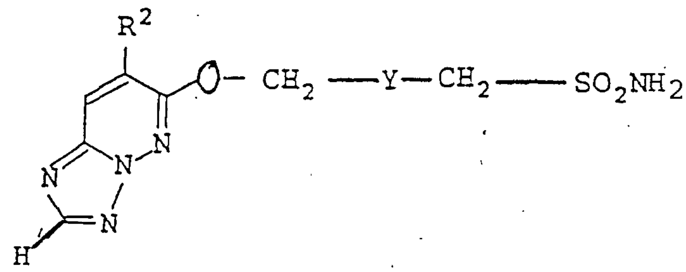

(I)

consisting of the following compounds:

6-(2,2-dimethyl-3-sulfamoyl-1-propoxy)-7-ethyl[1,2,4]triazolo [1,5-b]pyridazine

( $R^2$ : ethyl and Y : 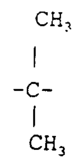 );

6-(2,2-dimethyl-3-sulfamoyl-1-propoxy)-7-n-propyl [1,2,4]triazolo[1,5-b]pyridazine

( $R^2$ : n-propyl and Y : 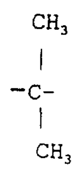 );

6-(2,2-dimethyl-3-sulfamoyl-1-propoxy)-7-isopropyl [1,2,4]triazolo[1,5-b]pyridazine

( $R^2$: isopropyl and Y: 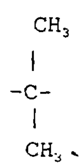 );

6-(2,2-diethyl-3-sulfamoyl-1-propoxy)-7-isopropyl [1,2,4]triazolo[1,5-b]pyridazine

( $R^2$: isopropyl and Y: 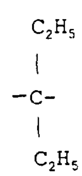 );

6-(2,2-dimethyl-3-sulfamoyl-1-propoxy)-7-isobutyl[1,2,4]triazolo[1,5-b]pyridazine

($R^2$: isobutyl and Y: 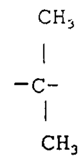 );

6-(2,2-dimethyl-3-sulfamoyl-1-propoxy)-7-t-butyl[1,2,4]triazolo[1,5-b]pyridazine

($R^2$: tertbutyl and Y: 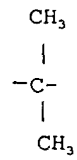 );

6-(2,2-dimethyl-3-sulfamoyl-1-propoxy)-7-n-hexyl[1,2,4]triazolo[1,5-b]pyridazine

($R^2$: n-hexyl and Y: 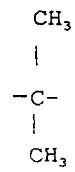 );

and
6-(2,2-dimethyl-3-sulfamoyl-1-propoxy)-7-cyclopentyl-[1,2,4]triazolo[1,5-b]pyridazine

($R^2$: cyclopentyl and Y : 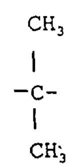 );

or a pharmaceutically acceptable salt thereof,

( 2 ) a process of producing a compound as described in (1) above which comprises reacting a compound of the formula[II:]

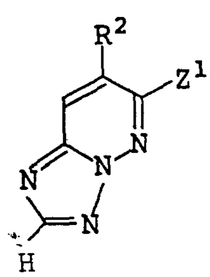

[II]

or a salt thereof, with a compound of the formula III:

$$Z^2O\text{-}CH_2\text{-}Y\text{-}CH_2\text{-}SO_2NH_2$$

or a salt thereof,
in which $Z^1$ and $Z^2$ are a leaving group upon mutual reaction; the other symbols are as described in (1) above,
( 3) a compound of the formula IV:

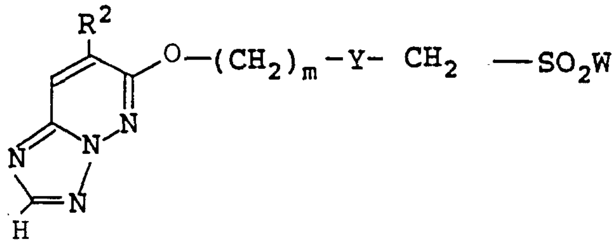

(IV)

wherein W is a leaving group; the other symbols are described in (1) above, or a salt thereof,
(4) a composition which comprises a compound as described in (1) above,
(5 ) a pharmaceutical composition which comprises a compound as described in (1) above,
(6) an anti-PAF composition, which comprises a compound as described in (1) above,
(7 ) a composition for inhibiting an eosinophil chemotaxis which comprises a compound as described in (1) above,
(8) an anti-asthmatic composition, which comprises a compound as described in (1) above,
(9) an anti-allergic composition, which comprises a compound as described in (1) above,
(10) a composition for preventing or treating allergic rhinitis, which comprises a compound as described in (1) above, and
(11) a composition for preventing or treating atopic dermatitis, which comprises a compound as described in (1) above.

Provided that compound [I] or a salt thereof contains an asymmetric carbon atom in its molecular structure, optical isomers and racemic mixtures are included in the scope of the present invention.
Preferable practical examples of the compound [I] or a salt thereof are as follows:

6-(2,2-dimethyl-3-sulfamoyl-1-propoxy)-7-ethyl[1,2,4]triazolo[1,5-b]pyridazine or a salt thereof,
6-(2,2-dimethyl-3-sulfamoyl-1-propoxy)-7-n-propyl[1,2,4]triazolo[1,5-b]pyridazine or a salt thereof,
6-(2,2-dimethyl-3-sulfamoyl-1-propoxy)-7-isopropyl[1,2,4]triazolo[1,5-b]pyridazine or a salt thereof,
6-(2,2-diethyl-3-sulfamoyl-1-propoxy)-7-isopropyl[1,2,4]triazolo[1,5-b]pyridazine or a salt thereof,
6-(2,2-dimethyl-3-sulfamoyl-1-propoxy)-7-isobutyl[1,2,4]triazolo[1,5-b]pyridazine or a salt thereof,
6-(2,2-dimethyl-3-sulfamoyl-1-propoxy)-7-tert-butyl[1,2,4]triazolo[1,5-b]pyridazine or a salt thereof,
6-(2,2-dimethyl-3-sulfamoyl-1-propoxy)-7-n-hexyl[1,2,4]triazolo[1,5.b]pyridazine

($R^2$: n-hexyl and Y: 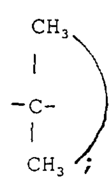 ;

and
6-(2,2-dimethyl-3-sulfamoyl-1-propoxy)-7-cyclopentyl-[1,2,4]triazolo[1,5.b]pyridazine

($R^2$: cyclopentyl and Y : 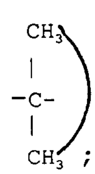 ;

or a pharmaceutically acceptable salt thereof.

The salt of compound [I] of the present invention is exemplified by salts with inorganic acids (e.g., hydrochloric acid, phosphoric acid, hydrobromic acid, sulfuric acid) and salts with organic acids (e.g., acetic acid, formic acid, propionic acid, fumaric acid, maleic acid, succinic acid, tartaric acid, citric acid, malic acid, oxalic acid, benzoic acid, methanesulfonic acid, benzenesulfonic acid), preferably physiologically acceptable acid-adduct salts. Provided that compound [I] of the present invention has an acidic group, such as -COOH, in a substituent thereof, it may form a salt with an inorganic base (e.g., an alkali metal or alkaline earth metal such as sodium, potassium, calcium or magnesium, or ammonia) or an organic base (e.g., a tri-$C_{1-3}$ alkylamine such as triethylamine).

Methods of producing the compound [I] of the present invention or salts thereof are described below.

The compound (I) of the present invention or a salt thereof can be synthesized by method (A), in which a compound of the formula II:

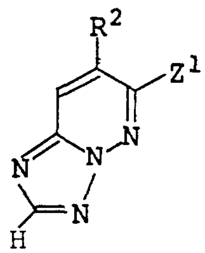

[II]

or a salt thereof, react with a compound of the formula III:

$$Z^2O\text{-}CH_2\text{-}Y\text{-}CH_2\text{-}SO_2NH_2 \qquad [III]$$

wherein the symbols are of the same meaning as defined above, or a salt thereof.

Examples of the leaving groups of $Z^1$ include halogen atoms (e.g., chlorine, bromine, iodine), $C_{6-10}$ arylsulfonyloxy groups (e.g., benzenesulfonyloxy, p-tolylsulfonyloxy) and $C_{1-4}$ alkylsulfonyloxy groups (e.g., methanesulfonyloxy).

The group of $Z^2$ which leaves the molecule upon reaction with $Z^1$ is exemplified by a hydrogen atom or an alkali metal such as sodium or potassium when X is an oxygen atom or a sulfur atom, or by an alkali metal such as sodium or potassium when X is -SO- or $-SO_2-$.

In this reaction, the compound [III] or a salt thereof is normally used at 1 to 5 mol, preferably 1 to 2 mol, per mol of the compound [II] or a salt thereof.

This condensation reaction is preferably carried out in the presence of a base. The base is exemplified by alkali metal hydrides such as sodium hydride and potassium hydride; alkali metal alkoxides such as sodium methoxide and sodium ethoxide; alkali metal hydroxides such as sodium hydroxide and potassium hydroxide; and carbonates such as sodium carbonate and potassium carbonate.

Also, this reaction can be carried out in an inert solvent, exemplified by alcohols such as methanol and ethanol; ethers such as dioxane and tetrahydrofuran; aromatic hydrocarbons such as benzene, toluene and xylene; nitriles such as acetonitrile; amides such as dimethylformamide and dimethylacetamide; and sulfoxides such as dimethyl sulfoxide.

Reaction temperature ranges usually from 10 to 200°C, preferably from 50 to 100°C. Reaction time ranges usually from 30 minutes to 24 hours, preferably from 1 to 6 hours.

The compound [I] of the present invention or a salt thereof can still also be synthesized by method ( ), in which a compound of the formula:

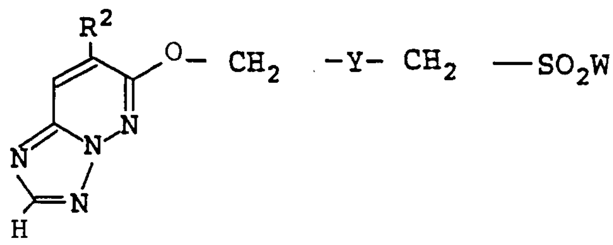

[IV]

wherein W represents a leaving group; the other symbols are of the same meaning as defined above, or a salt thereof, react with a compound of the formula:

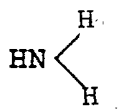

[V ]

wherein the symbols are of the same meaning as those given above, or a salt thereof.

Examples of the leaving groups of W include halogen atoms (e.g., chlorine, bromine, iodine), $C_{6-10}$ arylsulfonyloxy groups (e.g., benzenesulfonyloxy, p-tolylsulfonyloxy) and $C_{1-4}$ alkylsulfonyloxy groups (e.g., methanesulfonyloxy), preferably halogen atoms (e.g., chlorine, bromine, iodine).

In this reaction, the compound [V ] or a salt thereof is normally used at 1 to 5 mol, preferably 1 to 2 mol, per mol of the compound [IV] or a salt thereof.

This reaction can be carried out in an inert solvent exemplified by alcohols such as methanol and ethanol; ethers such as dioxane and tetrahydrofuran; aromatic hydrocarbons such as benzene, toluene and xylene; nitriles such as acetonitrile; amides such as dimethylformamide and dimethylacetamide; and sulfoxides such as dimethyl sulfoxide.

Reaction temperature ranges usually from -20 to 100°C, preferably from -10 to 50°C. Reaction time ranges usually from 30 minutes to 5 hours, preferably from 1 to 3 hours.

When the compound [I] is obtained in free form, it can be converted into a salt by a known method. When the compound [I] is obtained as a salt, it can be converted into a free form or another salt. The compound [I] or a salt thereof thus obtained can be isolated and purified by known means such as solvent extraction, pH adjustment, redistribution, salting out, crystallization, recrystallization and chromatography. When compound [I] or a salt thereof is obtained in the obtained in the mixture of optical isomers, it can be separated into the d- and 1-configurations by an ordinary means of optical resolution.

Hereinafter described are methods of producing starting compounds [II], [III], [IV] and [V], or salts thereof that are used to produce compound [I] or a salt thereof.

Salts of these compounds include salts with inorganic acids (e.g., hydrochloric acid, phosphoric acid, hydrobromic acid, sulfuric acid) and salts with organic acids (e.g., acetic acid, formic acid, propionic acid, fumaric acid, maleic acid, succinic acid, tartaric acid, citric acid, malic acid, oxalic acid, benzoic acid, methanesulfonic acid, benzenesulfonic acid). Provided that these compounds have an acidic group, such as -COOH, in a substituent thereof, it may form a salt with an inorganic base (e.g., an alkali metal or alkaline earth metal such as sodium, potassium, calcium or magnesium, or ammonia) or an organic base (e.g., a tri-$C_{1-3}$ alkylamine such as triethylamine).

The starting compound [II] or a salt thereof can, for example, be synthesized by the method described in the Journal of Organic Chemistry, Vol. 39, p. 2143 (1974) or a modification thereof.

The starting compound [III] or a salt thereof can, for example, be synthesized by the methods described in Chemische Berichte, Vol. 91, p. 2130 (1958), the Journal of Organic Chemistry, Vol. 52, p. 2162 (1987), and EP 381132/1990, or modifications thereof.

The starting compound [IV] or a salt thereof can, for example, be synthesized by (1) reacting a compound [II] or a salt thereof with a compound of the formula:

$$Z^2O—CH_2\text{-}Y\text{-}CH_2—SO_2W \qquad [VI]$$

wherein the symbols are of the same meaning as defined above, or (2) reacting a compound [VII]

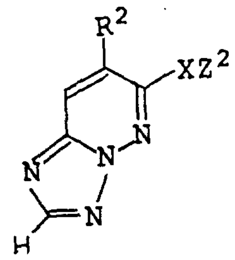

[VII]

or a salt thereof
with a compound of the formula:

$$Z^1 — CH_2 \text{-Y-} CH_2 — SO_2W \qquad [VIII]$$

wherein the symbols are of the same meaning as defined above.

In the reaction (1) above, the compound [VI ] is usually used in a ratio of 1 to 5 mol, preferably 1 to 2 mol, per mol of the compound [II] or a salt thereof. This reaction can be carried out in the same manner as the above-described reaction of the compound [II] or a salt thereof and the compound [III] or a salt thereof.

In the reaction (2) above, compound [VIII] is normally used in a ratio of 1 to 5 mol, preferably 1 to 2 mol, per mol of compound [VII] or a salt thereof. This reaction can be carried out in the same manner as the above-described reaction of the compound [VII] or a salt thereof and the compound [V] or a salt thereof.

The starting compound [V] or a salt thereof and starting compounds [VI ] and [VIII] can be synthesized by commonly known methods or modifications thereof.

Although the salts that may be formed by the above-described starting compounds are not subject to limitation, as long as the object of the present invention is accomplished, examples of salts include the same salts as the above-described salts that may be formed by compound [I].

Although these starting compounds or salts thereof thus obtained can be isolated and purified by known means such as solvent extraction, pH adjustment, redistribution, salting out, crystallization, recrystallization and chromatography, they may be used as starting materials for the next process, in the form of reaction mixture without purification.

When the starting compound used in each of the reactions for synthesizing the above-described desired compounds, the starting compound has an amino group, carboxyl group or hydroxyl group as a substituent, these substituents may have a protective group in common use in peptide chemistry etc.; the desired compound can be obtained by removing, as appropriate, the protective group after completion of the reaction.

Amino-protecting groups include, for example, formyl, $C_{1-6}$ alkylcarbonyl groups (e.g., acetyl, ethylcarbonyl), phenylcarbonyl group, $C_{1-6}$ alkyl-oxycarbonyl groups (e.g., methoxycarbonyl, ethoxycarbonyl), phenyloxycarbonyl group, $C_{7-10}$ aralkyl-carbonyl group (e.g., benzylcarbonyl), trityl group, phthaloyl group and N,N-dimethylaminomethylene group, which are optionally substituted. Examples of the substituents which these groups optionally have, include halogen atoms (e.g., fluorine, chlorine, bromine, iodine), $C_{1-6}$ alkyl-carbonyl groups (e.g., methylcarbonyl, ethylcarbonyl, butylcarbonyl) and nitro groups, the number of substituents being 1 to 3.

Examples of the carboxyl-protecting groups include $C_{1-6}$ alkyl groups (e.g., methyl, ethyl, n-propyl, isopropyl, n-butyl, tert-butyl), phenyl, trityl and silyl, which are optionally substituted. Examples of the substituents which these groups optionally have, include halogen atoms (e.g., fluorine, chlorine, bromine, iodine), formyl, $C_{1-6}$ alkyl-carbonyl groups (e.g., acetyl, ethylcarbonyl, butylcarbonyl) and nitro groups, the number of substituents being 1 to 3.

Examples of the hydroxyl-protecting groups include $C_{1-6}$ alkyl groups (e.g., methyl, ethyl, n-propyl, isopropyl, n-butyl, tert-butyl), phenyl, $C_{7-10}$ aralkyl groups (e.g., benzyl), formyl, $C_{1-6}$ alkyl-carbonyl groups (e.g., acetyl, ethylcarbonyl), phenyloxycarbonyl, benzoyl, $C_{7-10}$ aralkyl-carbonyl groups (e.g., benzylcarbonyl), pyranyl, furanyl and silyl, which are optionally substituted. Examples of substituents which these groups optionally have, include halogen atoms (e.g., fluorine, chlorine, bromine, iodine), $C_{1-6}$ alkyl groups (e.g., methyl, ethyl, n-propyl), phenyl, $C_{7-10}$ aralkyl groups (e.g., benzyl) and nitro groups, the number of substituents being 1 to 4.

Protecting groups can be removed by commonly known methods or modifications thereof, including treatments with acids, bases, reducing agents, ultraviolet rays, hydrazine, phenylhydrazine, sodium N-methyldithiocarbamate, tetrabutylammonium fluoride, palladium acetate etc.

The compound [I] or a salt thereof of this present invention can be safely used as an anti-asthmatic in mammals

(e.g., humans, mice, dogs, rats, bovines), because it exhibits excellent anti-allergic, anti-inflammatory, anti-PAF (platelet activating factor) and eosinophil chemotaxis inhibiting actions, with low toxicity (acute toxicity: $LD_{50}$ > 2 g/kg). The compound [I] or a salt thereof inhibit eosinophil chemotaxis and local eosinophil infiltration in allergic and inflammatory reactions. It is therefore useful as a therapeutic agent for diseases associated with eosinophil infiltration. Specifically, it can be used to treat or prevent eosinophilic diseases in the above-mentioned mammals, including allergic diseases such as urticaria, atopic dermatitis, allergic rhinitis and hypersensitive pneumonitis; dermal diseases such as eczema, herpetic dermatitis and psoriasis; and respiratory diseases such as eosinophilic pneumonia (PIE syndrome). Route of administration may be oral or non-oral.

The preparation for the present invention may contain as active ingredients pharmaceutical components other than the compound [I] or a salt thereof (e.g., anti-asthmatics, anti-allergic agents, anti-inflammatory agents, anti-bacterial agents, anti-fungal agents). Examples of these pharmaceutical components include anti-asthmatics (e.g., theophylline, procaterol, ketotifen, terfenadine, azelastine, seratrodast), anti-allergic agents (e.g., ketotifen, terfenadine, azelatine, epinastine), anti-inflammatory agents (e.g., dichlofenac sodium, ibprofen, indometacin), anti-bacterial agents (e.g., cefixime, cefdinir, ofloxacin, tosufloxacin), anti-fungal agents (e.g., fluconazole, itraconazole). These components are not subject to limitation, as long as the object of the present invention is accomplished, and may be used in appropriate mixing ratios. Useful dosage forms include tablets (including sugar-coated tablets and film-coated tablets), pills, capsules (including microcapsules), granules, fine subtilaes, powders, syrups, emulsions, suspensions, injectable preparations, inhalants and ointments. These preparations are prepared by conventional methods (e.g., methods described in the Pharmacopoeia of Japan). The content of the compound [I] or a salt thereof in the pharmaceutical preparation of the present invention varies with forms of the preparation, and it is usually 0.01 to 100 weight %, preferably 0.1 to 50 weight %, more preferably 0.5 to 20 weight %, relative to the whole weight of the pharmaceutical preparation.

Specifically, tablets can be produced by granulating a pharmaceutical as it is, or in a uniform mixture with excipients, binders, disintegrating agents and other appropriate additives, by an appropriate method, then adding lubricants etc., and subjecting the mixture to compressive shaping, or by subjecting to direct compressive shaping a pharmaceutical as it is, or in a uniform mixture with excipients, binders, disintegrating agents and other appropriate additives, or subjecting to compressive shaping previously prepared granules as it is, or in a uniform mixture with appropriate additives. These tablets may incorporate coloring agents, correctives etc. as necessary, and may be coated with appropriate coating agents.

Injectable preparations can be produced by dissolving, suspending or emulsifying a given amount of a pharmaceutical in an aqueous solvent such as water for injection, physiological saline or Ringer's solution, or a non-aqueous solvent such as a vegetable oil, and diluting to a given amount, or transferring a given amount of a pharmaceutical into an ampule for injection and sealing the ampule.

Useful carriers for oral preparations include substances in common use in pharmaceutical production, such as starch, mannitol, crystalline cellulose and carboxymethyl cellulose sodium. Useful carriers for injectable preparations include distilled water, physiological saline, glucose solutions and transfusions. Other additives in common use for pharmaceutical production can also be added, as appropriate.

Depending on patient age, body weight, symptoms, route and frequency of administration and other factors, the daily dose of these preparations is usually 0.1 to 100 mg/kg, preferably 1 to 50 mg/kg, and more preferably 1 to 10 mg/kg, based on daily dose of active ingredient (the compound [I] or a salt thereof), once or in two portions daily for each asthmatic adult.

## Best Mode for Carrying out the Invention

The present invention is hereinafter described in more detail by means of the following working examples, reference examples, preparation examples and experimental examples, which are not to be construed as limitative.

In the working examples below, the fraction containing the desired product was detected by observation via TLC (thin-layer chromatography). TLC was conducted on a TLC plate of Merck $60F_{254}$, using a UV detector.

Reference Example 1

Production of 3,6-dichloro-4-ethylpyridazine

To a suspension of 22.5 g of 3,6-dichloropyridazine, 12.7 g of silver nitrate, and 16.3 g of propionic acid in 250 ml of water was added a solution of 49.3 g of sulfuric acid in 250 ml of water at 50°C, followed by addition of 93 g of ammonium peroxodisulfate in 200 ml of water at 60°C for 20 minutes. The reaction mixture was heated at 70 to 75°C for 30 minutes. After cooling, the reaction mixture was adjusted to pH 7 with 25% ammonium hydroxide solution, and extracted with ethyl ether. The extract was washed with water and dried ($MgSO_4$) and the solvent was distilled off under reduced pressure. The residue was purified by silica gel column chromatography to provide 13.5 g of the title

compound.
NMR ($CDCl_3$) δ : 1.32 (3H,t,J=7Hz), 2.78(2H,q,J=7Hz), 7.39(1H,s).

Reference Example 2

Production of 3,6-dichloro-4-n-propylpyridazine

Using 16.6 g of 3,6-dichloropyridazine and 14.4 g of n-butric acid, the same reaction was conducted as in reference example 1 to produce 12.7 g of the title compound.
NMR ($CDCl_3$) δ : 1.10(3H,t,J=7Hz), 1.5-1.9(2H,m), 2.72(2H,t,J=7Hz), 7.37(1H,s)

Reference Example 3

Production of 3,6-dichloro-4-isobutylpyridazine

Using 22.3 g of 3,6-dichloropyridazine and 19.9 g of isovaleric acid, the same reaction was conducted as in reference example 1 to produce 15.0 g of the title compound.
NMR ($CDCl_3$) δ: 1.06(6H,d,J=7Hz), 1.8-2.2(1H,m), 2.60(2H,d,J=7Hz), 7.38(1H,s).

Reference Example 4

Production of 3,6-dichloro-4-n-hexylpyridazine

Using 11.7 g of 3,6-dichloropyridazine and 23 g of n-heptanoic acid, the same reaction was conducted as in reference example 1 to produce 14.4 g of the title compound.
NMR ($CDCl_3$) δ: 0.84-0.97(3H,m), 1.22-1.48(8H,m), 1.57-1.76(2H,m), 7.37(1H,s).

Reference Example 5

Production of 4-cyclopentyl-3,6-dichloropyridazine

Using 22.4 g of 3,6-dichloropyridazine and 25.1 g of cyclopentanecarboxylic acid, the same reaction was conducted as in reference example 1 to produce 28.7 g of the title compound.
NMR ($CDCl_3$) δ : 1.4-2.3(8H,m), 3.30(1H,m), 7.38(1H,s)

Reference Example 6

Production of 3-amino-6-chloro-5-ethylpyridazine

A mixture of 13.5 g of 3,6-dichloro-4-ethyl pyridazine in 150 ml of 25% ammonium hydroxide solution and 10 ml of ethanol was heated at 130 to 140°C in sealed tube for 22 hours. After cooling, the solvent was distilled off. Water was added to the residue and resulting crystals were collected by filtration and washed with water and ethyl ether to provide 6.96 g of the title compound.
NMR ($d_6$-DMSO) δ : 1.16(3H,t,J=7Hz), 2.54(2H,q,J=7Hz), 6.48(2H,s), 6.73(1H,s)

Reference Example 7

Production of 3-amino-6-chloro-5-n-propylpyridazine

Using 12.7 g of 3,6-dichloro-5-n-propylpyridazine, 150 ml of 25% ammonium hydroxide solution, and 5 ml of ethanol, the same reaction was conducted as in reference example 6 to produce 6.8 g of the title compound.
NMR ($CDCl_3$) δ: 1.00(3H,t,J=7Hz), 1.57-1.76(2H,m), 2.59(2H,t,J=7Hz), 4.80(2H,br.s), 6.60(1H,s).

Reference Example 8

Production of 3-amino-6-chloro-5-isopropylpyridazine

Using 21.7 g of 3,6-dichloro-4-isopropylpyridazine, 170 ml of 25% ammonium hydroxide solution, and 10 ml

of ethanol, the same reaction was conducted as in reference example 6 to produce 11.1 g of the title compound.
m.p. 164-165°C
NMR ($CDCl_3$) δ: 1.26(6H,d,J=7Hz), 3.16(1H,m), 4.89(2H,br.s), 6.65(1H,s).

Reference Example 9

Production of 3-amino-6-chloro-5-isobutylpyridazine

Using 15.0 g of 3,6-dichloro-4-isobutylpyridazine, 180 ml of 25% ammonium hydroxide solution, and 5 ml of ethanol, the same reaction was conducted as in reference example 6 to produce 7.3 g of the title compound.
m.p. 122-123°C
NMR ($CDCl_3$) δ: 0.96(6H,d,J=7Hz), 1.8-2.2(1H,m), 2.48(2H,d,J=7Hz), 4.88(2H,br.s), 6.38(1H,s).

Reference Example 10

Production of 3-amino-5-t-butyl-6-chloropyridazine

Using 31.1 g of 4-t-butyl-3,6-dichloropyridazine, 150 ml of 25% ammonium hydroxide solution, and 15 ml of ethanol, the same reaction was conducted as in reference example 6 to produce 20.8 g of the title compound.
m.p. 185-188°C
NMR ($CDCl_3$) δ: 1.44(9H,s), 4.88(2H,br.s), 6.74(1H,s). Reference Example 11

Production of 3-amino-6-chloro-5-n-hexylpyridazine

Using 14.4 g of 3,6-dichloro-4-n-hexylpyridazine, 150 ml of 25% ammonium hydroxide solution, and 150 ml of ethanol, the same reaction was conducted as in reference example 6 to produce 6.2 g of the title compound.
m.p. 97-98°C
NMR ($CDCl_3$) δ: 0.84-1.02(3H,m), 1.20-1.48(8H,m), 1.54-1.71(2H,m), 4.97(2H,br.s), 6.62(1H,s).

Reference Example 12

Production of 3-amino-6-chloro-5-cyclopentylpyridazine

Using 28.7 g of 4-cyclopentyl-3,6-dichloropyridazine, 210 ml of 25% ammonium hydroxide solution, and 70 ml of ethanol, the same reaction was conducted as in reference example 6 to produce 15.5 g of the title compound.
NMR ($CDCl_3$) δ: 1.4-2.3(8H,m), 3.20(1H,m), 4.84(2H,br.s), 6.65(1H,s)

Reference Example 13

Production of N-(6-chloro-5-ethylpyridazine-3-yl)formamide oxime

To a suspension of 4.6 g of 3-amino-6-chloro-5-ethylpyridazine in 58 ml of toluene was added 6.2 ml of dimethylformamide dimethyl acetal and the reaction mixture was refluxed for 2 hours. The solvent was distilled off under reduced pressure. The residue was purified by silica gel column chromatography and elution was carried out with ethyl acetate-methanol (10:1). The fractions containing the objective compound were pooled and concentrated. The residue was dissolved in methanol (40 ml) and 1.8 g of hydroxylamine hydrochloride was added to the solution and stirred at room temperature for 2 hours. The resulting crystals were collected by filteration and washed with a small amount of methanol to provide 4.40 g of the title compound.
m.p. 175-178°C

| Elemental analysis for $C_7H_9N_4OCl$ | | | |
|---|---|---|---|
| Calcd.(%): | C, 41.91; | H, 4.52; | N, 27.93 |
| Found (%): | C, 41.73; | H, 4.65; | N, 27.69 |

Reference Example 14

Production of N-(6-chloro-5-n-propylpyridazine-3-yl) formamide oxime

Using 6.8 g of 3-amino-6-chloro-5-n-propylpyridazine, the same reaction was conducted as in reference example 13 to produce 4.7 g of the title compound.
NMR ($d_6$-DMSO) δ: 0.95(3H,t,J=7Hz), 1.5-1.8(2H,m), 2.57(2H,t,J=7Hz), 7.36(1H,s) 7.91(1H,d,J=10Hz), 9.65(1H,d, J=10Hz), 10.40(1H,s).

Reference Example 15

Production of N-(6-chloro-5-isopropylpyridazine-3-yl) formamide oxime

Using 8.6 g of 3-amino-6-chloro-5-isopropylpyridazine, the same reaction was conducted as in reference example 13 to produce 10.6 g of the title compound.
m.p. 170-171°C

| Elemental analysis for $C_8H_{11}N_4OCl$ | | | |
|---|---|---|---|
| Calcd.(%): | C, 44.76; | H, 5.17; | N, 26.10 |
| Found (%): | C, 44.62; | H, 5.02; | N, 26.01 |

Reference Example 16

Production of N-(6-chloro-5-isobutylpyridazine-3-yl) formamide oxime

Using 7.0 g of 3-amino-6-chloro-5-isobutylpyridazine, the same reaction was conducted as in reference example 13 to produce 4.9 g of the title compound.
m.p. 155-158°C

| Elemental analysis for $C_9H_{13}N_4OCl \cdot H_2O$ | | | |
|---|---|---|---|
| Calcd.(%): | C, 43.82; | H, 6.13; | N, 22.71 |
| Found (%): | C, 43.23; | H, 6.21; | N, 23.00 |

Reference Example 17

Production of N-(5-t-butyl-6-chloropyridazine-3-yl) formamide oxime

Using 11.1 g of 3-amino-5-t-butyl-6-chloropyridazine, the same reaction as conducted as in reference example 13 to produce 12.0 g of the title compound
m.p. 221-224°C

| Elemental analysis for $C_9H_{13}N_4OCl$ | | | |
|---|---|---|---|
| Calcd.(%): | C, 47.27; | H, 5.73; | N, 24.50 |
| Found (%): | C, 47.10; | H, 5.48; | N, 24.68 |

Reference Example 18

Production of N-(6-chloro-5-n-hexylpyridazine-3-yl) formamide oxime

Using 6.0 g of 3-amino-6-chloro-5-n-hexylpyridazine, the same reaction as conducted as in reference example 13 to produce 5.7 g of the title compound.
m.p. 131-133°C

| Elemental analysis for $C_{11}H_{17}N_4OCl$ | | | |
|---|---|---|---|
| Calcd.(%): | C, 51.46; | H, 6.67; | N, 21.82 |
| Found (%): | C, 51.15; | H, 6.87; | N, 21.59 |

Reference Example 19

Production of N-(6-chloro-5-cyclopentylpyridazine-3-yl) formamide oxime

Using 8.9 g of 3-amino-6-chloro-5-cyclopentylpyridazine, the same reaction as conducted as in reference example 13 to produce 8.05 g of the title compound.
m.p. 207-208°C

| Elemental analysis for $C_{10}H_{13}N_4OCl$ | | | |
|---|---|---|---|
| Calcd.(%): | C, 49.90; | H, 5.44; | N, 23.28 |
| Found (%): | C, 49.85; | H, 5.60; | N, 23.31 |

Reference Example 20

Production of 6-chloro-7-ethyl[1,2,4]triazolo[1,5-b]pyridazine

A mixture of 4.02 g of N-(6-chloro-5-ethylpyridazine-3-yl)formamide oxime and 25 g of polypyhosphoric acid was heated at 110 to 115°C for 1 hour in oil bath. After cooling, the reaction mixture was poured into ice water and extracted with dichloromethane. The extract was washed with water and dried ($MgSO_4$) and the solvent was distilled off under reduced pressure. Hexane was added to the residue and the resulting crystals were collected by filtration, to provide 2.33 g of the title compound.
m.p. 84-85°C

| Elemental analysis for $C_7H_7ClN_4$ | | | |
|---|---|---|---|
| Calcd.(%): | C, 46.04; | H, 3.86; | N, 30.68 |
| Found (%): | C, 46.00; | H, 3.79; | N, 30.59 |

Reference Example 21

Production of 6-chloro-7-n-propyl[1,2,4]triazolo[1,5-b]pyridazine

Using 4.7 g of N-(6-chloro-5-n-propylpyridazine-3-yl)formamide oxime and 23 g of polyphosphoric acid, the same reaction was conducted as in reference example 20 to produce 3.1 g of the title compound.
m.p. 61-62°C

| Elemental analysis for $C_8H_9ClN_4$ | | | |
|---|---|---|---|
| Calcd.(%): | C, 48.87; | H, 4.61; | N, 28.49 |
| Found (%): | C, 48.87; | H, 4.55; | N, 28.55 |

Reference Example 22

Production of 6-chloro-7-isopropyl[1,2,4]triazolo[1,5-b]pyridazine

Using 20.04 g of N-(6-chloro-5-isopropylpyridazine-3-yl)formamide oxime and 97 g of polyphosphoric acid, the same reaction was conducted as in reference example 20 to produce 12.7 g of the title compound.
m.p. 53-54°C

| Elemental analysis for $C_8H_9ClN_4$ | | | |
|---|---|---|---|
| Calcd.(%): | C, 48.87; | H, 4.61; | N, 28.49 |
| Found (%): | C, 48.85; | H, 4.55; | N, 28.48 |

Reference Example 23

Production of 6-chloro-7-isobutyl[1,2,4]triazolo[1,5-b]pyridazine

Using 4.6 g of N-(6-chloro-5-isobutylpyridazine-3-yl)formamide oxime and 22 g of polyphosphoric acid, the same reaction was conducted as in reference example 20 to produce 2.2 g of the title compound.
m.p. 60-62°C

| Elemental analysis for $C_9H_{11}ClN_4$ | | | |
|---|---|---|---|
| Calcd.(%): | C, 51.31; | H, 5.26; | N, 26.60 |
| Found (%): | C, 51.33; | H, 5.05; | N, 26.71 |

Reference Example 24

Production of 7-t-butyl-6-chloro[1,2,4]triazolo[1,5-b]pyridazine

Using 8.0 g of N-(5-t-butyl-6-chloropyridazine-3-yl)formamide oxime and 38 g of polyphosphoric acid, the same reaction was conducted as in reference example 20 to produce 5.13 g of the title compound.
m.p.110-112°C

| Elemental analysis for $C_9H_{11}ClN_4$ | | | |
|---|---|---|---|
| Calcd.(%): | C, 51.31; | H, 5.26; | N, 26.60 |
| Found (%): | C, 51.15; | H, 5.15; | N, 26.66 |

Reference Example 25

Production of 6-chloro-7-n-hexyl[1,2,4]triazolo[1,5-b]pyridazine

Using 5.5 g of N-(6-chloro-5-n-hexylpyridazine-3-yl)formamide oxime and 22 g of polyphosphoric acid, the same reaction was conducted as in reference example 20 to produce 2.2 g of the title compound.
m.p. 52-53°C

| Elemental analysis for $C_{11}H_{15}ClN_4$ | | | |
|---|---|---|---|
| Calcd.(%): | C, 55.35; | H, 6.33; | N, 23.47 |
| Found (%): | C, 55.25; | H, 6.12; | N, 23.52 |

Reference Example 26

Production of 6-chloro-7-cyclopentyl[1,2,4]triazolo[1,5-b]pyridazine

Using 7.76 g of N-(6-chloro-5-cyclopentylpyridazine-3-yl)formamide oxime and 40 g of polyphosphoric acid, the same reaction was conducted as in reference example 20 to produce 5.51 g of the title compound.
m.p. 57-59°C

| Elemental analysis for $C_{10}H_{11}ClN_4$ | | | |
|---|---|---|---|
| Calcd.(%): | C, 53.94; | H, 4.98; | N, 25.16 |
| Found (%): | C, 53.88; | H, 4.97; | N, 25.07 |

Example 1

Production of 6-(2,2-dimethyl-3-sulfamoyl-l-propoxy)-7-ethyl[1,2,4]triazolo[1,5-b]pyridazine

To a solution of 0.836 g of 3-hydroxy-2,2-dimethylpropane-1-sulfonamide in 25 ml of tetrahydrofuran was added 0.42 g of 60% sodium hydride in oil and the mixture was refluxed for 40 minutes. After cooling, to the reaction mixture was added 0.913 g of 6-chloro-7-ethyl[1,2,4]triazolo[1,5-b]pyridazine and the mixture was refluxed with stirring for 2 hours. After cooling, the reaction mixture was poured into water, adjusted to pH6 with 1N-hydrochloric acid, and extracted with ethyl acetate-tetrahydrofuran (1:1). The extract was washed with saturated aqueous solution of sodium chloride and dried ($MgSO_4$) and the solvent was distilled off under reduced pressure. The residue was purified by silica gel column chromatography and elution was carried out with dichloromethane-ethyl acetate-methanol (10:10:1). The fractions containing the objective compound were pooled and the resulting crystals were recrystallized from acetone-water to provide 0.787 g of the title compound.
m.p. 222-224°C

| Elemental analysis for $C_{12}H_{19}N_5O_3S$ | | | |
|---|---|---|---|
| Calcd.(%): | C, 45.99; | H, 6.11; | N, 22.35 |
| Found (%): | C, 45.87; | H, 6.14; | N, 20.33 |

Example 2

Production of 6-(2,2-dimethyl-3-sulfamoyl-1-propoxy)-7-n-propyl[1,2,4]triazolo[1,5-b]pyridazine

Using 0.90 g of 3-hydroxy-2,2-dimethyl-1-propanesulfonamide and 1.0 g of 6-chloro-7-n-propyl[1,2,4]triazolo [1,5-b]pyridazine, the same reaction was conducted as in example 1 to produce 0.77 g of the title compound.
m.p. 196-197°C

| Elemental analysis for $C_{13}H_{21}N_5O_3S \cdot H_2O$ | | | |
|---|---|---|---|
| Calcd.(%): | C, 45.20; | H, 6.70; | N, 20.27 |
| Found (%): | C, 45.68; | H, 6.39; | N, 20.55 |

Example 3

Production of 6-(2,2-dimethyl-3-sulfamoyl-1-propoxy)-7-isopropyl[1,2,4]triazolo[1,5-b]pyridazine

To a solution of 0.669 g of 3-hydroxy-2,2-dimethyl-1-propanesulfonamide in 30 ml of tetrahydrofuran was added 0.336 g of 60% sodium hydride in oil and the mixture was refluxed for 1 hour. After cooling, to the reaction mixture was added 0.748 g of 6-chloro-7-isopropyl[1,2,4]triazolo[1,5-b]pyridazine and the mixture was refluxed with stirring for 4 hours. After cooling, the reaction mixture was poured into ice water, adjusted to pH6 with IN-hydrochloric acid, and extracted with ethyl acetate-tetrahydrofuran (1:1). The extract was washed with saturated aqueous solution of sodium chloride and dried ($MgSO_4$) and the solvent was distilled off under reduced pressure. The residue was purified by silica gel column chromatography and elution was carried out with dichloromethane-ethyl acetate-methanol (10:10:1). The fractions containing the objective compound were pooled and the resulting crystals were recrystallized from acetone-water to provide 1.10 g of the title compound.
m.p. 197-198°C

| Elemental analysis for $C_{13}H_{21}N_5O_3S$ | | | |
|---|---|---|---|
| Calcd.(%): | C, 47.69; | H, 6.46; | N, 21.39 |
| Found (%): | C, 47.84; | H, 6.60; | N, 21.33 |

Example 4

Production of 6-(2,2-diethyl-3-sulfamoyl-1-propoxy)-7-isopropyl[1,2,4]triazolo[1,5-b]pyridazine

Using 0.586 g of 3-hydroxy-2,2-diethylpropane-1-sulfonamide and 0.561 g of 6-chloro-7-isopropyl[1,2,4]triazolo[1,5-b]pyridazine, the same reaction was conducted as in example 1 to produce 0.967 g of the title compound.
m.p. 190-192°C

| Elemental analysis for $C_{15}H_{22}N_5O_3S$ | | | |
|---|---|---|---|
| Calcd.(%): | C, 50.68; | H, 7.09; | N, 19.70 |
| Found (%): | C, 50.90; | H, 7.30; | N, 19.42 |

Example 5

Production of 6-(2,2-dimethyl-3-sulfamoyl-1-propoxy)-7-isobutyl[1,2,4]triazolo[1,5-b]pyridazine

Using 0.84 g of 3-hydroxy-2,2-dimethylpropane-1-sulfonamide and 1.0 g of 6-chloro-7-isobutyl[1,2,4]triazolo[1,5-b]pyridazine, the same reaction was conducted as in example 1 to produce 0.63 g of the title compound.
m.p. 132-135°C

| Elemental analysis for $C_{14}H_{23}N_5O_3S \cdot H_2O$ | | | |
|---|---|---|---|
| Calcd.(%): | C, 46.78; | H, 6.45; | N, 19.98 |
| Found (%): | C, 46.91; | H, 6.40; | N, 19.79 |

Example 6

Production of 6-(2,2-dimethyl-3-sulfamoyl-1-propoxy)-7-t-butyl[1,2,4]triazolo[1.5-b]pyridazine

Using 1.0 g of 3-hydroxy-2,2-dimethylpropane-1-sulfonamide and 1.2 g of 7-t-butyl-6-chloro[1,2,4]triazolo[1,5-b]pyridazine, the same reaction was conducted as in example 1 to produce 1.13 g of the title compound.
m.p. 168-170°C

| Elemental analysis for $C_{14}H_{23}N_5O_3S$ | | | |
|---|---|---|---|
| Calcd.(%): | C, 49.25; | H, 6.79; | N, 20.51 |
| Found (%): | C, 49.12; | H, 6.69; | N, 20.81 |

Example 7

Production of 6-(2,2-dimethyl-3-sulfamoyl-1-propoxy)-7-n-hexyl[1,2,4]triazolo[1,5-b]pyridazine

Using 0.74 g of 3-hydroxy-2,2-dimethylpropane-1-sulfonamide and 1.0 g of 6-chloro-7-n-hexyl[1,2,4]triazolo[1,5-b]pyridazine, the same reaction was conducted as in example 1 to produce 1.5 g of the title compound.
m.p. 150-151°C

| Elemental analysis for $C_{16}H_{27}N_5O_3S$ | | | |
|---|---|---|---|
| Calcd.(%): | C, 52.01; | H, 7.37; | N, 18.95 |
| Found (%): | C, 52.00; | H, 7.32; | N, 19.15 |

Example 8

Production of 6-(2,2-dimethyl-3-sulfamoyl-1-propoxy)-7-cyclopentyl[1,2,4]triazolo[1,5-b]pyridazine

Using 1.01 g of 3-hydroxy-2,2-dimethylpropane-1-sulfonamide and 1.27 g of 6-chloro-7-cyclopentyl[1,2,4]triazolo[1,5-b]pyridazine, the same reaction was conducted as in example 1 to produce 1.56 g of the title compound.
m.p. 170-172°C

| Elemental analysis for $C_{15}H_{23}N_5O_3S \cdot 0.5H_2O$ | | | |
|---|---|---|---|
| Calcd.(%): | C, 49.71; | H, 6.67; | N, 19.32 |

(continued)

| Elemental analysis for $C_{15}H_{23}N_5O_3S \cdot 0.5H_2O$ | | | |
|---|---|---|---|
| Found (%): | C, 49.65; | H, 6.69; | N, 19.48 |

Experimental Example 1

The results of a pharmacological test of the compound [I] or salt thereof according to the invention are shown below.

Effect on platelet activating factor (PAF)-induced bronchoconstriction in guinea pig

Male Hartley guinea pigs (body weights about 500 g) were used. Bronchoconstriction induced by PAF (1 µg/kg i.v.) in guinea pigs was measured according to the method of Konzett-Roessler. The guinea pig immobilized in the dorsal position, tracheotomy was anesthetized with urethane (1.5 g/kg i.v.) and the trachea was connected through a cannula to a respirator. The side branch of the tracheal cannula was connected to a broncho transducer (Model 7020, Ugobasile). The volume of overflow air was recorded on a rectigraph (Recte-Hori-8S, San-ei Sokki) through the brancho transducer. After guinea pig was given gallamine (1 mg/kg i.v.), the guinea pig was treated by PAF (1 µg/kg). The drug suspended in a 5% solution of gum arabic was administered orally in a dose of 3 mg/kg or 1 mg/kg one hour before PAF treatment. The results are presented in Table 1.

Table 1

| Effect on PAF-induced Bronchoconstriction | | |
|---|---|---|
| Example Number | % Inhibition of PAF-induced bronchoconstriction | |
| | 1 mg/kg, P.O. | 3 mg/kg, P.O. |
| 1 | 70 | |
| 2 | 67 | |
| 3 | 72 | |
| 4 | 60 | |
| 6 | 43 | 83 |
| Reference compound | | 25 |
| Reference compound: Compound of Example 3 in EP562439 6-(2,2-dimethyl-3-sulfamoyl-1-propoxy)-7-methyl [1,2,4]triazolo[1,5-b]pyridazine | | |

From Table 1, it is seen that the desired compound [I] or a salt thereof of the present invention possesses greater inhibitory effect than that of the compound having a methyl group at 7-position of the triazolopyridazine skeleton.

Experimental Example 2

1) Preparation of a guinea pig eosinophil suspension

Male Hartley guinea pigs (body weights approx. 300 g) were intraperitoneally treated with 2 ml of horse serum (Whittaker Bioproducts) once a week for 8 consecutive weeks. Forty-eight (48) hours after the last treatment, 75 ml of physiological saline was infused into the peritoneal cavity and the lavage was collected and centrifuged at 400 G (1500 rpm) for 5 minutes. The pellet was suspended in 5 ml of Percoll discontinuous density, gradients (specific gravity d=1.07), layered on top of the Percoll suspensions (specific gravity d=1.112:5 ml, d=1.095:10 ml, d=1.090:10 ml, d=1.085:7 ml) and centrifuged (18°C) at 1000 G (2200 rpm) for 30 minutes. The cell layer formed in the boundary zone between the d=1.112 and d=1.095 layers was collected. The collected cell sediment was subjected to hypotonic treatment (suspended in water for 30 seconds) to remove the contaminant erythrocytes.

The sediment was washed three times with Hanks solution containing 10 mM Hepes (Dojin Kagaku) (Hanks-Hepes) and suspended in Hanks-Hepes containing 2% (w/v) human serum albumin (Wako Pure Chemical) (Hanks-Hepes-HSA) at a concentration of $5.56 \times 10^6$ celles/ml. The eosinophil purity was 92-96% and the viability of eosinophils was not less than 98%.

2) Chemotaxis inhibition assay

To a 24-well petri dish, which serves as a lower chamber, 600 μl of $LTB_4$ (final concentration $10^{-8}$ M, Cascade Biochemical Ltd.) in suspension in Hanks-Hepes-HSA solution, was transferred, followed by incubation at 37°C for 30 minutes in carbon dioxide incubator. Separately, 200 μl ($5 \times 10^6$ cells/ml) of eosinophil suspension, previously incubated at 37°C for 15 minutes in a constant-temperature chamber, was added to Chemotaxicell (polycarbonate membrane, pore size 3 μm, thickness 10 μm), which serves as an upper chamber, after the upper chamber was attached to the 24-well petri dish. After 2 hours of reaction in the carbon dioxide incubator, the Chemotaxicell was removed; 60 μl of a 2% (w/v) solution of EDTA in physiological saline was added to the liquid in the lower chamber. After the mixture was ice cooled, the cells migrating into the lower chamber liquid were counted using a blood cell counter (Coulter Counter (trade name)). The test drug, dissolved in dimethyl formamide (DMF), was added to both the upper and lower chambers to a final concentration of $10^{-5}$ M.

Equation 1

Chemotaxis inhibition rate =

$$\left(1 - \frac{\text{Sum of chemotactic cells in the presence of the drug}}{\text{Sum of chemotactic cells in the absence of the drug}}\right) \times 100$$

Table 2

| Inhibitory Effect on $LTB_4$-induced Chemotactic of Guinea Pig Eosinophils | |
|---|---|
| Compound | Chemotaxis Inhibition Rate (%) |
| Example 1 | 35 |
| Example 2 | 24 |
| Example 4 | 23 |
| Example 5 | 27 |
| Example 6 | 38 |
| Example 7 | 24 |
| Reference compound | 12 |
| Reference compound: Same as used in Experimental Example 1 | |

From Table 2, it is seen that the desired compound [I] of the present invention or a salt thereof has more potent inhibitory activity against eosinophil chemotaxis than that of the compound having a methyl group at the 7-position in the triazolopyridazine skeleton.

| Preparation Example 1 | | |
|---|---|---|
| (1) | Compound of Example 3 | 10.0 mg |
| (2) | Lactose | 60.0 mg |
| (3) | Corn starch | 35.0 mg |
| (4) | Gelatin | 3.0 mg |
| (5) | Magnesium stearate | 2.0 mg |

A mixture of 10.0 mg of the compound obtained in Example 3, 60.0 mg of lactose and 35.0 mg of corn starch was granulated through a sieve of 1 mm mesh, using 0.03 ml of a 10% aqueous solution of gelatin (containing 3.0 mg of gelatin), after which it was dried at 40°C and again sieved. The resulting granules were mixed with 2.0 mg of magnesium stearate, followed by compression. The resulting core tablets were coated with a sugar coat, using an aqueous

suspension of sucrose, titanium dioxide, talc and gum arabic. The coated tablets were polished with beeswax to yield finished coated tablets.

| Preparation Example 2 | | |
|---|---|---|
| (1) | Compound of Example 3 | 10.0 mg |
| (2) | Lactose | 70.0 mg |
| (3) | Corn starch | 50.0 mg |
| (4) | Soluble starch | 7.0 mg |
| (5) | Magnesium stearate | 3.0 mg |

10.0 mg of the compound obtained in Example 3 and 3.0 mg of magnesium stearate were mixed and granulated, using 0.07 ml of an aqueous solution of soluble starch (containing 7.0 mg of soluble starch). The resulting granules were dried and mixed with 70.0 mg of lactose and 50.0 mg of corn starch, followed by compression, to yield tablets.

| Preparation Example 3 | | |
|---|---|---|
| (1) | Compound of Example 3 | 5.0 mg |
| (2) | Sodium chloride | 20.0 mg |
| (3) | Distilled water was added to reach a total quantity of 2 ml. | |

5.0 mg of the compound obtained in Example 3 and 20.0 mg of sodium chloride were dissolved in distilled water, and diluted with water to reach a total quantity of 2.0 ml. The resulting solution was filtered and aseptically packed in a 2 ml ampule, which was sterilized and sealed to yield a solution for injection.

Industrial Applicability

Possessing excellent anti-PAF and eosinophil chemotaxis inhibiting activities, the compound [I] or a salt thereof of the present invention is useful as a prophylactic/therapeutic drug for asthma, allergic rhinitis, atopic dermatitis etc.

| | | | |
|---|---|---|---|
| Calcd.(%): | C, 52.01; | H, 7.37; | N, 18.95 |
| Found (%): | C, 52.00; | H, 7.32; | N, 19.15 |

Example 8

Production of 6-(2,2-dimethyl-3-sulfamoyl-1-propoxy)-7-cyclopentyl[1,2,4]triazolo[1,5-b]pyridazine

Using 1.01 g of 3-hydroxy-2,2-dimethylpropane-1-sulfonamide and 1.27 g of 6-chloro-7-cyclopentyl[1,2,4]triazolo[1,5-b]pyridazine, the same reaction was conducted as in example 1 to produce 1.56 g of the title compound. m.p. 170-172°C

| Elemental analysis for $C_{15}H_{23}N_5O_3S{\cdot}0.5H_2O$ | | | |
|---|---|---|---|
| Calcd.(%): | C, 49.71; | H, 6.67; | N, 19.32 |
| Found (%): | C, 49.65; | H, 6.69; | N, 19.48 |

Example 9

Production of 7-isopropyl-6-(3-sulfamoyl-1-propoxy)[1,2,4]triazolo[1,5-b]pyridazine

168 mg of 60% oily sodium hydride was suspended in 25 ml of tetrahydrofuran; 279 mg of 3-hydroxypropane-1-sulfonamide was added, followed by refluxing for 40 minutes. After the mixture was cooled, 394 mg of 6-chloro-7-isopropyl[1,2,4]triazolo[1,5-b]pyridazine was added, followed by stirring at room temperature for 20 hours. After ice water was added, 5 N hydrochloric acid was added to bring the mixture to pH 5. The solution was then saturated with sodium chloride and extracted with tetrahydrofuran; the extract was washed with saturated saline and dried over magnesium sulfate. After the dry product was concentrated under reduced pressure, the resulting crystal was washed with

ethyl ether and recrystallized from 95% ethanol to yield 414 mg of the title compound.
m.p. 190-191°C

| Elemental analysis for $C_{11}H_{17}N_5O_3S$: | | | |
|---|---|---|---|
| Calcd. (%): | C, 44.14; | H, 5.72; | N, 23.39 |
| Found (%): | C, 44.12; | H, 5.77; | N, 22.92 |

Example 10

Production of 6-(2,2-dimethyl-3-sulfamoyl-1-propoxy)-7-isopropyl-2-methyl[1,2,4]triazolo[1,5-b]pyridazine

252 mg of 60% oily sodium hydride was suspended in 30 ml of tetrahydrofuran; 527 mg of 3-hydroxy-2,2-dimethylpropane-1-sulfonamide was added, followed by refluxing for 1 hour. After the mixture was cooled, 632 mg of 6-chloro-7-isopropyl-2-methyl[1,2,4]triazolo[1,5-b]pyridazine was added, followed by thermal refluxing for 6 hours. After the mixture was cooled, ice water was added; 5 N hydrochloric acid was added to bring the mixture to pH 5. The solution was then saturated with sodium chloride and extracted with ethyl acetate-tetrahydrofuran (2:1); the extract was washed with saturated saline and dried over magnesium sulfate. After the extract was concentrated under reduced pressure, the resulting residue was subjected to silica gel column chromatography and eluted with dichloromethane-ethyl acetate-methanol (10:10:1). The desired fraction was collected; the resulting crystal was recrystallized from 95% ethanol to yield 746 mg of the title compound.
m.p. 196-199°C

| Elemental analysis for $C_{14}H_{23}N_5O_3S$: | | | |
|---|---|---|---|
| Calcd. (%): | C, 49.25; | H, 6.79; | N, 20.51 |
| Found (%): | C, 49.18; | H, 6.76; | N, 20.44 |

Example 11

Production of 6-(2,2-diethyl-3-sulfamoyl-1-propoxy)-7-t-butyl[1,2,4]triazolo[1,5-b]pyridazine

380 mg of 60% oily sodium hydride was suspended in 30 ml of tetrahydrofuran; 980 mg of 3-hydroxy-2,2-diethyl-propane-l-sulfonamide was added, followed by refluxing for 1 hour. After the mixture was cooled, 1.0 g of 7-t-butyl-6-chloro[1,2,4]triazolo[1,5-b]pyridazine was added, followed by refluxing for 3 hours. After the mixture was cooled, ice water was added; 5 N hydrochlo-ric acid was added to bring the mixture to pH 4. The solution was then saturated with sodium chloride and extracted with tetrahydrofuran; the extract was washed with saturated saline and dried over magnesium sulfate. After the extract was concentrated under reduced pressure, the resulting residue was purified by silica gel column chromatography and eluted with dichloromethane-methanol (30:1). The desired fraction was collected to yield 720 mg of the title compound.
m.p. 172-174°C

| Elemental analysis for $C_{16}H_{27}N_5O_3S$: | | | |
|---|---|---|---|
| Calcd. (%): | C, 52.01; | H, 7.37; | N, 18.95 |
| Found (%): | C, 51.86; | H, 7.31; | N, 18.90 |

Experimental Example 1

The results of a pharmacological test of the compound [I] or salt thereof according to the invention are shown below.

Effect on platelte activating factor (PAF)-induced bronchoconstriction in guinea pig

Male Hartley guinea pigs (body weights about 500 g) were used. Bronchoconstriction induced by PAF (1 μg/kg i.v.) in guinea pigs was measured according to the method of Konzett-Roessler. The guinea pig immobilized in the dorsal position, tracheotomy was anesthetized with urethane (1.5 g/kg i.v.) and the trachea was connected through a cannula to a respirator. The side branch of the tracheal cannula was connected to a broncho transducer (Model 7020,

Ugobasile). The volume of overflow air was recorded on a rectigraph (Recte-Hori-8S, San-ei Sokki) through the brancho transducer. After guinea pig was given gallamine (1 mg/kg i.v.), the guinea pig was treated by PAF (1 µg/kg). The drug suspended in a 5% solution of gum arabic was administered orally in a dose of 3 mg/kg or 1 mg/kg one hour before PAF treatment. The results are presented in Table 1.

Table 1

| Effect on PAF-induced Bronchoconstriction | | |
|---|---|---|
| Example Number | % Inhibition of PAF-induced bronchoconstriction | |
| | 1 mg/kg, P.O. | 3 mg/kg, P.O. |
| 1 | 70 | |
| 2 | 67 | |
| 3 | 72 | |
| 4 | 60 | |
| 6 | 43 | 83 |
| Reference compound | | 25 |
| Reference compound: Compound of Example 3 in EP562439 6-(2,2-dimethyl-3-sulfamoyl-1-propoxy)-7-methyl [1,2,4]triazolo[1,5-b]pyridazine | | |

From Table 1, it is seen that the desired compound [I] or a salt thereof of the present invention possesses greater inhibitory effect than that of the compound having a methyl group at 7-position of the triazolopyridazine skeleton.

Experimental Example 2

1) Preparation of a guinea pig eosinophil suspension

Male Hartley guinea pigs (body weights approx. 300 g) were intraperitoneally treated with 2 ml of horse serum (Whittaker Bioproducts) once a week for 8 consecutive weeks. Forty-eight (48) hours after the last treatment, 75 ml of physiological saline was infused into the peritoneal cavity and the lavage was collected and centrifuged at 400 G (1500 rpm) for 5 minutes. The pellet was suspended in 5 ml of Percoll discontinuous density, gradients (specific gravity d=1.07), layered on top of the Percoll suspensions (specific gravity d=1.112:5 ml, d=1.095:10 ml, d=1.090:10 ml, d=1.085:7 ml) and centrifuged (18°C) at 1000 G (2200 rpm) for 30 minutes. The cell layer formed in the boundary zone between the d=1.112 and d=1.095 layers was collected. The collected cell sediment was subjected to hypotonic treatment (suspended in water for 30 seconds) to remove the contaminant erythrocytes.

The sediment was washed three times with Hanks solution containing 10 mM Hepes (Dojin Kagaku) (Hanks-Hepes) and suspended in Hanks-Hepes containing 2% (w/v) human serum albumin (Wako Pure Chemical) (Hanks-Hepes-HSA) at a concentration of 5.56 x $10^6$ celles/ml. The eosinophil purity was 92-96% and the viability of eosinophils was not less than 98%.

2) Chemotaxis inhibition assay

To a 24-well petri dish, which serves as a lower chamber, 600 µl of $LTB_4$ (final concentration $10^{-8}$ M, Cascade Biochemical Ltd.) in suspension in Hanks-Hepes-HSA solution, was transferred, followed by incubation at 37°C for 30 minutes in carbon dioxide incubator. Separately, 200 µl ($5 \times 10^6$ cells/ml) of eosinophil suspension, previously incubated at 37°C for 15 minutes in a constant-temperature chamber, was added to Chemotaxicell (polycarbonate membrane, pore size 3 µm, thickness 10 µm), which serves as an upper chamber, after the upper chamber was attached to the 24-well petri dish. After 2 hours of reaction in the carbon dioxide incubator, the Chemotaxicell was removed; 60 µl of a 2% (w/v) solution of EDTA in physiological saline was added to the liquid in the lower chamber. After the mixture was ice cooled, the cells migrating into the lower chamber liquid were counted using a blood cell counter (Coulter Counter (trade name)). The test drug, dissolved in dimethyl formamide (DMF), was added to both the upper and lower chambers to a final concentration of $10^{-5}$ M.

Equation 1

$$\text{Chemotaxis inhibition rate} = \left(1 - \frac{\text{Sum of chemotactic cells in the presence of the drug}}{\text{Sum of chemotactic cells in the absence of the drug}}\right) \times 100$$

Table 2

| Inhibitory Effect on $LTB_4$-induced Chemotactic of Guinea Pig Eosinophils | |
|---|---|
| Compound | Chemotaxis Inhibition Rate (%) |
| Example 1 | 35 |
| Example 2 | 24 |
| Example 4 | 23 |
| Example 5 | 27 |
| Example 6 | 38 |
| Example 7 | 24 |
| Reference compound | 12 |
| Reference compound: Same as used in Experimental Example 1 | |

From Table 2, it is seen that the desired compound [I] of the present invention or a salt thereof has more potent inhibitory activity against eosinophil chemotaxis than that of the compound having a methyl group at the 7-position in the triazolopyridazine skeleton.

| Preparation Example 1 | | |
|---|---|---|
| (1) | Compound of Example 3 | 10.0 mg |
| (2) | Lactose | 60.0 mg |
| (3) | Corn starch | 35.0 mg |
| (4) | Gelatin | 3.0 mg |
| (5) | Magnesium stearate | 2.0 mg |

A mixture of 10.0 mg of the compound obtained in Example 3, 60.0 mg of lactose and 35.0 mg of corn starch was granulated through a sieve of 1 mm mesh, using 0.03 ml of a 10% aqueous solution of gelatin (containing 3.0 mg of gelatin), after which it was dried at 40°C and again sieved. The resulting granules were mixed with 2.0 mg of magnesium stearate, followed by compression. The resulting core tablets were coated with a sugar coat, using an aqueous suspension of sucrose, titanium dioxide, talc and gum arabic. The coated tablets were polished with beeswax to yield finished coated tablets.

| Preparation Example 2 | | |
|---|---|---|
| (1) | Compound of Example 3 | 10.0 mg |
| (2) | Lactose | 70.0 mg |
| (3) | Corn starch | 50.0 mg |
| (4) | Soluble starch | 7.0 mg |
| (5) | Magnesium stearate | 3.0 mg |

10.0 mg of the compound obtained in Example 3 and 3.0 mg of magnesium stearate were mixed and granulated, using 0.07 ml of an aqueous solution of soluble starch (containing 7.0 mg of soluble starch). The resulting granules were dried and mixed with 70.0 mg of lactose and 50.0 mg of corn starch, followed by compression, to yield tablets.

| Preparation Example 3 | | |
|---|---|---|
| (1) | Compound of Example 3 | 5.0 mg |
| (2) | Sodium chloride | 20.0 mg |
| (3) | Distilled water was added to reach a total quantity of 2 ml. | |

5.0 mg of the compound obtained in Example 3 and 20.0 mg of sodium chloride were dissolved in distilled water, and diluted with water to reach a total quantity of 2.0 ml. The resulting solution was filtered and aseptically packed in a 2 ml ampule, which was sterilized and sealed to yield a solution for injection.

Industrial Applicability

Possessing excellent anti-PAF and eosinophil chemotaxis inhibiting activities, the compound [I] or a salt thereof of the present invention is useful as a prophylactic/therapeutic drug for asthma, allergic rhinitis, atopic dermatitis etc.

## Claims

1. Compounds of the formula I:

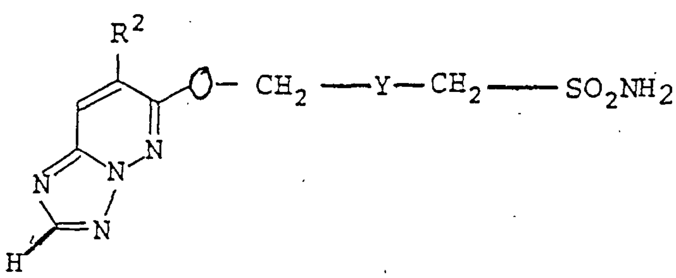

(I)

consisting of the following compounds:

6-(2,2-dimethyl-3-sulfamoyl-1-propoxy)-7-ethyl[1,2,4]triazolo [1,5-b]pyridazine

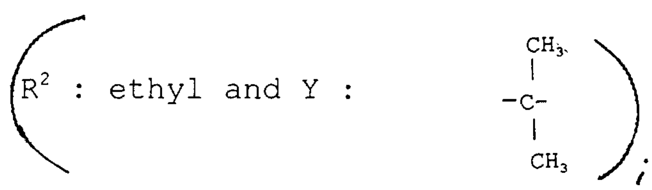

6-(2,2-dimethyl-3-sulfamoyl-1-propoxy)-7-n-propyl [1,2,4]triazolo[1,5-b]pyridazine

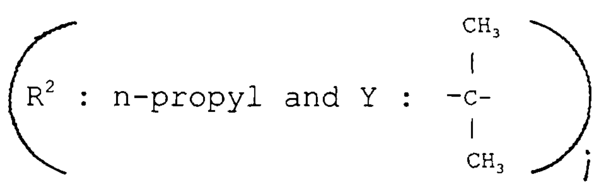

6-(2,2-dimethyl-3-sulfamoyl-1-propoxy)-7-isopropyl [1,2,4]triazolo[1,5-b]pyridazine

($R^2$: isopropyl and Y: 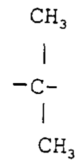 )

6-(2,2-diethyl-3-sulfamoyl-1-propoxy)-7-isopropyl [1,2,4]triazolo[1,5-b]pyridazine

($R^2$: isopropyl and Y: 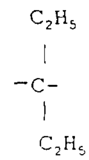 );

6-(2,2-dimethyl-3-sulfamoyl-1-propoxy)-7-isobutyl[1,2,4]triazolo[1,5-b]pyridazine

($R^2$: isobutyl and Y: 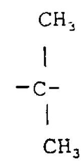 );

6-(2,2-dimethyl-3-sulfamoyl-1-propoxy)-7-t-butyl[1,2,4]triazolo[1,5-b]pyridazine

($R^2$: tertbutyl and Y: 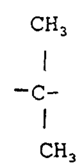 );

6-(2,2-dimethyl-3-sulfamoyl-1-propoxy)-7-n-hexyl[1,2,4]triazolo[1,5-b]pyridazine

($R^2$: n-hexyl and Y: 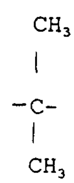 );

and
6-(2,2-dimethyl-3-sulfamoyl-1-propoxy)-7-cyclopentyl-[1,2,4]triazolo[1,5-b]pyridazine

($R^2$: cyclopentyl and Y : 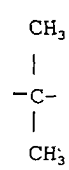 );

or a pharmaceutically acceptable salt thereof.

**2.** A compound of formula I , which is selected from:

6-(2,2-dimethyl-3-sulfamoyl-1-propoxy)-7-ethyl-[1,2,4]triazolo[1,5-b]pyridazine or a pharmaceutically acceptable salt thereof

$R^2$: ethyl and Y:

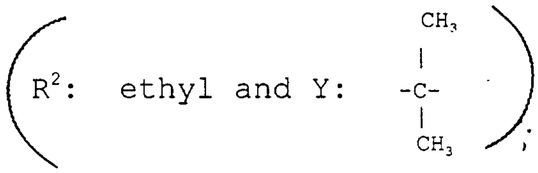

6-(2,2-dimethyl-3-sulfamoyl-1-propoxy)-7-n-propyl [1,2,4]triazolo[1,5-b]pyridazine or a pharmaceutically acceptable salt thereof

$R^2$: n-propyl and Y:

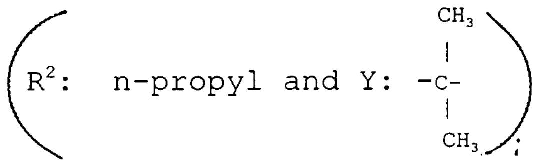

6-(2,2-dimethyl-3-sulfamoyl-1-propoxy)-7-isopropyl [1,2,4]triazolo[1,5-b]pyridazine or a pharmaceutically acceptable salt thereof

$R^2$ : isopropyl and Y:

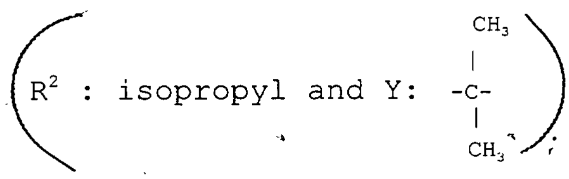

6-(2,2-diethyl-3-sulfamoyl-1-propoxy)-7-isopropyl [1,2,4]triazolo[1,5-b]pyridazine or a pharmaceutically acceptable salt thereof

$R^2$: isopropyl and Y:

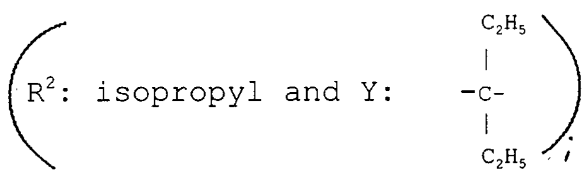

6-(2,2-dimethyl-3-sulfamoyl-1-propoxy)-7-isobutyl[1,2,4]triazolo[1,5-b]pyridazine or a pharmaceutically acceptable salt thereof

$R^2$: isobutyl and Y:

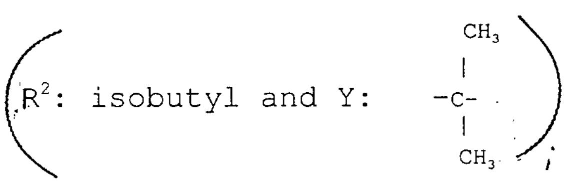

6-(2,2-dimethyl-3-sulfamoyl-1-propoxy)-7-tert-butyl[1,2,4]triazolo[1,5-b]pyridazine or a pharmaceutically ac-

ceptable salt thereof

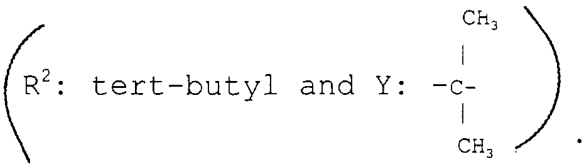


3. 6-(2,2-dimethyl-3-sulfamoyl-1-propoxy)-7-isopropyl[1,2,4]triazolo[1,5-b]pyridazine

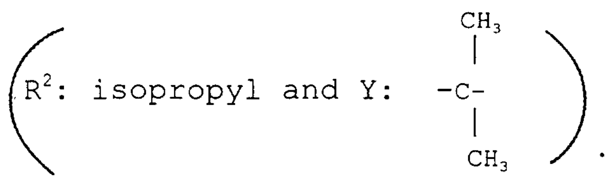


4. A process of producing a compound of claim 1, 2, or 3 which comprises reacting a compound of the formula II:

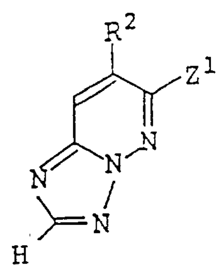

(II)

or a pharmaceutically acceptable salt thereof, with a compound of the formula III:

$$Z^2O\text{-}CH_2\text{-}Y\text{-}CH_2\text{-}SO_2NH_2 \quad \text{(III)}$$

or a salt thereof, in which
$Z^1$ and $Z^2$ are a leaving group upon mutual reaction, the other symbols are as defined in claim 1, 2 or 3.

5. A compound of the formula IV:

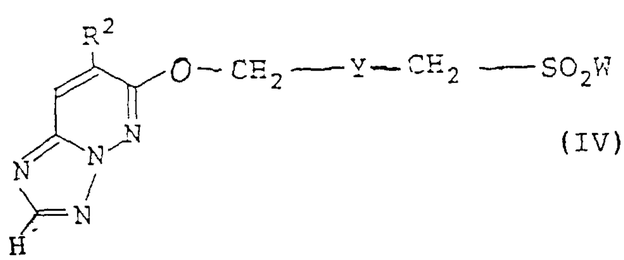

(IV)

wherein W is a leaving group, the other symbols are as defined above, or a salt thereof.

6. A composition which comprises a compound of claim 1, 2, or 3 or a pharmaceutically acceptable salt.

7. A pharmaceutical composition which comprises a compound of claim 1, 2, or 3 or a pharmaceutically acceptable salt.

8. An anti-PAF composition, which comprises a compound of claim 1, 2, or 3 or a pharmaceutically acceptable salt.

9. A pharmaceutical composition for inhibiting an eosinophil chemotaxis, which comprises a compound of claim 1, 2,or 3 or a pharmaceutically acceptable salt.

10. An anti-asthmatic composition, which comprises a compound of claim 1, 2, or 3 or a pharmaceutically acceptable salt.

11. An anti-allergic composition, which comprises a compound of claim 1, 2, or 3 or a pharmaceutically acceptable salt.

12. , A composition for preventing or treating allergic rhinitis, which comprises a compound of claim 1, 2, or 3 or a pharmaceutically acceptable salt.

13. A composition for preventing or treating atopic dermatitis, which comprises a compound of claim 1, 2,or 3 or a pharmaceutically acceptable salt.

14. The composition of claim 6 which is a tablet, an inhalant or a fine subtilae.

15. The composition of claim 7 which is a tablet, an inhalant or a fine subtilae.

16. The composition of claim 8 which is a tablet, an inhalant or a fine subtilae.

17. The composition of claim 9 which is a tablet, an inhalant or a fine subtilae.

18. The composition of claim 10 which is a tablet, an inhalant or a fine subtilae.

19. , The composition of claim 11 which is a tablet, an inhalant or a fine subtilae.

20. The composition of claim 12 which is a tablet, an inhalant or a fine subtilae.

21. The composition of claim 13 which is a tablet, an inhalant or a fine subtilae.

22. Use of a compound of claim 1, 2, or 3 or a pharmaceutically acceptable salt for manufacturing an anti-PAF composition.

23. Use of a compound of claim 1, 2, or 3 or a pharmaceutically acceptable salt for manufacturing a pharmaceutical composition for inhibiting an eosinophil chemotaxis.

24. Use of a compound of claim 1, 2, or 3 or a pharmaceutically acceptable salt for manufacturing an anti-asthmatic composition.

25. Use of a compound of claim 1, 2, or 3 or a pharmaceutically acceptable salt for manufacturing an anti-allergic composition.

26. Use of a compound of claim 1, 2, or 3 or a pharmaceutically acceptable salt for manufacturing a composition for preventing or treating allergic rhinitis.

27. Use of a compound of claim 1, 2, or 3 or a pharmaceutically acceptable salt for manufacturing a composition for preventing or treating atopic dermatitis.

## Patentansprüche

1. Verbindungen der Formel I:

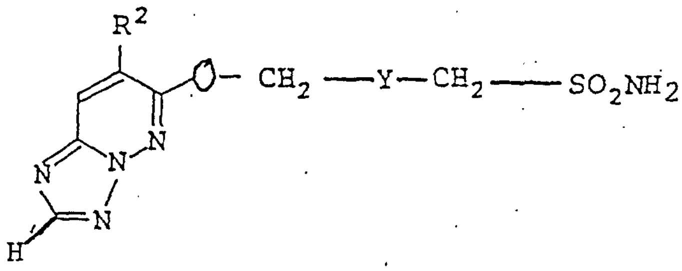

(I)

die aus den folgenden Verbindungen bestehen:

6-(2,2-Dimethyl-3-sulfamoyl-1-propoxy)-7-ethyl[1,2,4]triazolo[1,5-b]pyridazin

($R^2$ = Ethyl und Y = 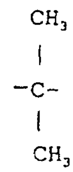 );

6-(2,2-Dimethyl-3-sulfamoyl-1-propoxy)-7-n-propyl[1,2,4]triazolo[1,5-b]pyridazin

($R^2$ = n-Propyl und Y = 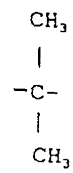 );

6-(2,2-Dimethyl-3-sulfamoyl-1-propoxy)-7-isopropyl[1,2,4]triazolo[1,5-b]pyridazin

($R^2$ = Isopropyl und Y = 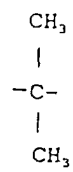 );

6-(2,2-Diethyl-3-sulfamoyl-1-propoxy)-7-isopropyl[1,2,4]triazolo[1,5-b]pyridazin

($R^2$ = Isopropyl und Y = 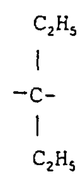 );

6-(2,2-Dimethyl-3-sulfamoyl-1-propoxy)-7-isobutyl[1,2,4]triazolo[1,5-b]pyridazin

($R^2$ = Isobutyl und Y = 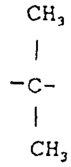 );

6-(2,2-Dimethyl-3-sulfamoyl-1-propoxy)-7-t-butyl[1,2,4]triazolo[1,5-b]pyridazin

($R^2$ = tert-Butyl und Y = 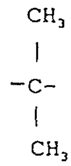 );

6-(2,2-Dimethyl-3-sulfamoyl-1-propoxy)-7-n-hexyl[1,2,4]triazolo[1,5-b]pyridazin

($R^2$ = n-Hexyl und Y = 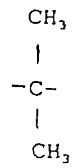 );

und

6-(2,2-Dimethyl-3-sulfamoyl-1-propoxy)-7-cyclopentyl[1,2,4]triazolo[1,5-b]pyridazin

($R^2$ = Cyclopentyl und Y = 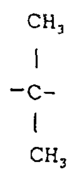 );

oder ein pharmazeutisch annehmbares Salz davon.

**2.** Verbindung der Formel I, die ausgewählt ist aus:

6-(2,2-Dimethyl-3-sulfamoyl-1-propoxy)-7-ethyl[1,2,4]triazolo[1,5-b]pyridazin oder einem pharmazeutisch annehmbaren Salz davon

($R^2$ = Ethyl und Y = 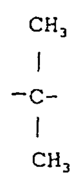 );

6-(2,2-Dimethyl-3-sulfamoyl-1-propoxy)-7-n-propyl[1,2,4]triazolo[1,5-b]pyridazin oder einem pharmazeutisch annehmbaren Salz davon

(R$^2$ = n-Propyl und Y = 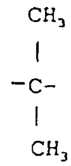 );

6-(2,2-Dimethyl-3-sulfamoyl-1-propoxy)-7-isopropyl[1,2,4]triazolo[1,5-b]pyridazin oder einem pharmazeutisch annehmbaren Salz davon

(R$^2$ = Isopropyl und Y = 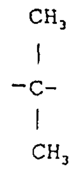 );

6-(2,2-Diethyl-3-sulfamoyl-1-propoxy)-7-isopropyl[1,2,4]triazolo[1,5-b]pyridazin oder einem pharmazeutisch annehmbaren Salz davon

(R$^2$ = Isopropyl und Y = 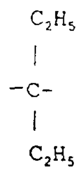 );

6-(2,2-Dimethyl-3-sulfamoyl-1-propoxy)-7-isobutyl[1,2,4]triazolo[1,5-b]pyridazin oder einem pharmazeutisch annehmbaren Salz davon

(R$^2$ = Isobutyl und Y = 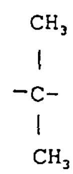 );

6-(2,2-Dimethyl-3-sulfamoyl-1-propoxy)-7-t-butyl[1,2,4]triazolo[1,5-b]pyridazin oder einem pharmazeutisch annehmbaren Salz davon

(R$^2$ = tert-Butyl und Y = 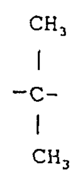 ).

**3.** 6-(2,2-Dimethyl-3-sulfamoyl-1-propoxy)-7-isopropyl[1,2,4]triazolo[1,5-b]pyridazin oder einem pharmazeutisch annehmbaren Salz davon

($R^2$ = Isopropyl und Y = 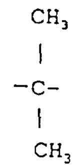 ).

**4.** Verfahren zur Herstellung einer Verbindung gemäß Anspruch 1, 2 oder 3, umfassend das Umsetzen einer Verbindung der Formel II

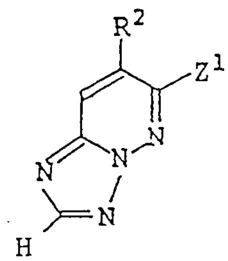

(II)

oder eines pharmazeutisch annehmbaren Salzes davon mit einer Verbindung der Formel III

$$Z^2O\text{-}CH_2\text{-}Y\text{-}CH_2\text{-}SO_2NH_2 \qquad \text{(III)}$$

oder einem Salz davon, wobei
$Z^1$ und $Z^2$ Abgangsgruppen bei einer Reaktion miteinander sind und die anderen Symbole wie in Anspruch 1, 2 oder 3 definiert sind.

**5.** Verbindung der Formel IV:

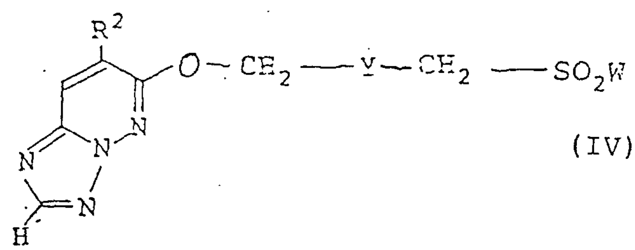

(IV)

wobei W eine Abgangsgruppe ist und die anderen Symbole wie oben definiert sind, oder ein Salz davon.

**6.** Zusammensetzung, die eine Verbindung gemäß Anspruch 1, 2 oder 3 oder ein pharmazeutisch annehmbares Salz davon umfasst.

**7.** Pharmazeutische Zusammensetzung, die eine Verbindung gemäß Anspruch 1, 2 oder 3 oder ein pharmazeutisch annehmbares Salz davon umfasst.

**8.** Anti-PAF-Zusammensetzung, die eine Verbindung gemäß Anspruch 1, 2 oder 3 oder ein pharmazeutisch annehmbares Salz davon umfasst.

**9.** Pharmazeutische Zusammensetzung zur Hemmung einer Eosinophilen-Chemotaxis, die eine Verbindung gemäß

Anspruch 1, 2 oder 3 oder ein pharmazeutisch annehmbares Salz davon umfasst.

**10.** Antiasthmatische Zusammensetzung, die eine Verbindung gemäß Anspruch 1, 2 oder 3 oder ein pharmazeutisch annehmbares Salz davon umfasst.

**11.** Antiallergische Zusammensetzung, die eine Verbindung gemäß Anspruch 1,2 oder 3 oder ein pharmazeutisch annehmbares Salz davon umfasst.

**12.** Zusammensetzung zur Prävention oder Behandlung von allergischer Rhinitis, die eine Verbindung gemäß Anspruch 1, 2 oder 3 oder ein pharmazeutisch annehmbares Salz davon umfasst.

**13.** Zusammensetzung zur Prävention oder Behandlung von atopischer Dermatitis, die eine Verbindung gemäß Anspruch 1, 2 oder 3 oder ein pharmazeutisch annehmbares Salz davon umfasst.

**14.** Zusammensetzung gemäß Anspruch 6, bei der es sich um eine Tablette, ein Inhalans oder ein Feingranulat handelt.

**15.** Zusammensetzung gemäß Anspruch 7, bei der es sich um eine Tablette, ein Inhalans oder ein Feingranulat handelt.

**16.** Zusammensetzung gemäß Anspruch 8, bei der es sich um eine Tablette, ein Inhalans oder ein Feingranulat handelt.

**17.** Zusammensetzung gemäß Anspruch 9, bei der es sich um eine Tablette, ein Inhalans oder ein Feingranulat handelt.

**18.** Zusammensetzung gemäß Anspruch 10, bei der es sich um eine Tablette, ein Inhalans oder ein Feingranulat handelt.

**19.** Zusammensetzung gemäß Anspruch 11, bei der es sich um eine Tablette, ein Inhalans oder ein Feingranulat handelt.

**20.** Zusammensetzung gemäß Anspruch 12, bei der es sich um eine Tablette, ein Inhalans oder ein Feingranulat handelt.

**21.** Zusammensetzung gemäß Anspruch 13, bei der es sich um eine Tablette, ein Inhalans oder ein Feingranulat handelt.

**22.** Verwendung einer Verbindung gemäß Anspruch 1, 2 oder 3 oder eines pharmazeutisch annehmbaren Salzes davon zur Herstellung einer Anti-PAF-Zusammensetzung.

**23.** Verwendung einer Verbindung gemäß Anspruch 1, 2 oder 3 oder eines pharmazeutisch annehmbaren Salzes davon zur Herstellung einer pharmazeutischen Zusammensetzung zur Hemmung einer Eosinophilen-Chemotaxis.

**24.** Verwendung einer Verbindung gemäß Anspruch 1, 2 oder 3 oder eines pharmazeutisch annehmbaren Salzes davon zur Herstellung einer anti-asthmatischen Zusammensetzung.

**25.** Verwendung einer Verbindung gemäß Anspruch 1, 2 oder 3 oder eines pharmazeutisch annehmbaren Salzes davon zur Herstellung einer antiallergischen Zusammensetzung.

**26.** Verwendung einer Verbindung gemäß Anspruch 1, 2 oder 3 oder eines pharmazeutisch annehmbaren Salzes davon zur Herstellung einer Zusammensetzung zur Prävention oder Behandlung von allergischer Rhinitis.

**27.** Verwendung einer Verbindung gemäß Anspruch 1, 2 oder 3 oder eines pharmazeutisch annehmbaren Salzes davon zur Herstellung einer Zusammensetzung zur Prävention oder Behandlung von atopischer Dermatitis.

## Revendications

**1.** Composés de formule (I) :

(I)

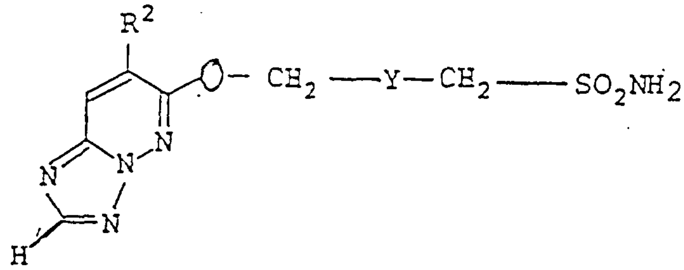

choisis parmi les composés suivants :

- 6-(2,2-diméthyl-3-sulfamoyl-1-propoxy)-7-éthyl[1,2,4]triazolo-[1,5.b]pyridazine ($R^2$ : éthyle, Y : $-C(CH_3)_2-$),
- 6-(2,2-diméthyl-3-sulfamoyl-1-propoxy)-7-n-propyl[1,2,4]triazolo-[1,5.b]pyridazine ($R^2$ : n-propyle, Y : $-C(CH_3)_2-$),
- 6-(2,2-diméthyl-3-sulfamoyl-1-propoxy)-7-isopropyl[1,2,4]triazolo-[1,5.b]pyridazine ($R^2$ : isopropyle, Y : $-C(CH_3)_2-$),
- 6-(2,2-diéthyl-3-sulfamoyl-1-propoxy)-7-isopropyl[1,2,4]triazolo-[1,5.b]pyridazine ($R^2$ : isopropyle, Y : $-C(C_2H_5)_2-$),
- 6-(2,2-diméthyl-3-sulfamoyl-1-propoxy)-7-isobutyl[1,2,4]triazolo-[1,5.b]pyridazine ($R^2$ : isobutyle, Y : $-C(CH_3)_2-$),
- 6-(2,2-diméthyl-3-sulfamoyl-1-propoxy)-7-tert-butyl[1,2,4]triazolo-[1,5.b]pyridazine ($R^2$ : tert-butyle, Y : $-C(CH_3)_2-$),
- 6-(2,2-diméthyl-3-sulfamoyl-1-propoxy)-7-n-hexyl[1,2,4]triazolo-[1,5.b]pyridazine ($R^2$ : n-hexyle, Y : $-C(CH_3)_2-$), et
- 6-(2,2-diméthyl-3-sulfamoyl-1-propoxy)-7-cyclopentyl[1,2,4]triazolo-[1,5.b]pyridazine ($R^2$ : cyclopentyle, Y : $-C(CH_3)_2-$)

ou des sels pharmaceutiquement acceptables de ces composés.

**2.** Composé de formule (Ia) qui est choisi parmi la 6-(2,2-diméthyl-3-sulfamoyl-1-propoxy)-7-éthyl[1,2,4]triazolo [1,5.b]pyridazine ($R^2$ : éthyle, Y : $-C(CH_3)_2-$) ou un sel pharmaceutiquement acceptable de celle-ci, la 6-(2,2-diméthyl-3-sulfamoyl-1-propoxy)-7-n-propyl[1,2,4]triazolo-[1,5.b]pyridazine ($R^2$ : n-propyle, Y : $-C(CH_3)_2-$) ou un sel pharmaceutiquement acceptable de celle-ci, la 6-(2,2-diméthyl-3-sulfamoyl-1-propoxy)-7-isopropyl[1,2,4]triazolo-[1,5.b]pyridazine ($R^2$ : isopropyle, Y : $-C(CH_3)_2-$) ou un sel pharmaceutiquement acceptable de celle-ci, la 6-(2,2-diéthyl-3-sulfamoyl-1-propoxy)-7-isopropyl[1,2,4]triazolo-[1,5.b]pyridazine ($R^2$ : isopropyle, Y : $-C(C_2H_5)_2-$) ou un sel pharmaceutiquement acceptable de celle-ci, la 6-(2,2-diméthyl-3-sulfamoyl-1-propoxy)-7-isobutyl[1,2,4] triazolo-[1,5.b]pyridazine ($R^2$ : isobutyle, Y : $-C(CH_3)_2-$) ou un sel pharmaceutiquement acceptable de celle-ci, et la 6-(2,2-diméthyl-3-sulfamoyl-1-propoxy)-7-tert-butyl[1,2,4]triazolo-[1,5.b]pyridazine ($R^2$ : tert-butyle, Y : $-C(CH_3)_2-$) ou un sel pharmaceutiquement acceptable de celle-ci.

**3.** 6-(2,2-diméthyl-3-sulfamoyl-1-propoxy)-7-isopropyl-[1,2,4]triazolo-[1,5.b]pyridazine ($R^2$ : isopropyle, Y : $-C(CH_3)_2-$).

**4.** Procédé de préparation d'un composé selon la revendication 1, 2 ou 3, comprenant le fait de faire réagir un composé de formule (II) ou un sel pharmaceutiquement acceptable d'un tel composé

(II) 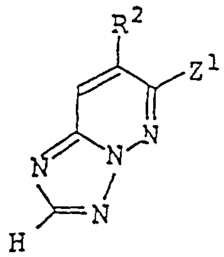

avec un composé de formule (III)

(III) $Z^2O\text{-}CH_2\text{-}Y\text{-}CH_2\text{-}SO_2NH_2$

ou un sel d'un tel composé, formules dans lesquelles $Z^1$ et $Z^2$ représentent des groupes partant lorsque les composés réagissent l'un avec l'autre, les autres symboles étant définis comme dans la revendication 1, 2 ou 3.

**5.** Composé de formule (IV)

(IV) 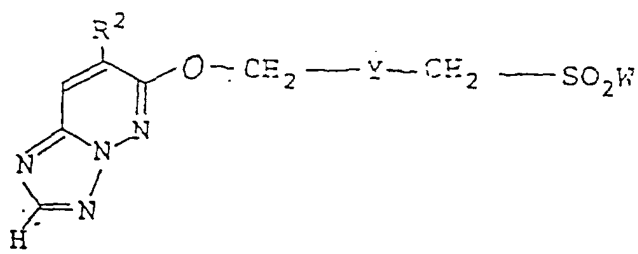

dans laquelle W représente un groupe partant, les autres symboles ayant la signification indiquée ci-dessus, ou sel d'un tel composé.

**6.** Composition contenant un composé selon une des revendications 1, 2 ou 3 ou un sel pharmaceutiquement acceptable de celui-ci.

**7.** Composition pharmaceutique contenant un composé selon une des revendications 1, 2 ou 3 ou un sel pharmaceutiquement acceptable de celui-ci.

**8.** Composition anti-PAF contenant un composé selon une des revendications 1, 2 ou 3 ou un sel pharmaceutiquement acceptable de celui-ci.

**9.** Composition pharmaceutique pour inhiber la chimiotaxie d'éosinophiles contenant un composé selon une des revendications 1, 2 ou 3 ou un sel pharmaceutiquement acceptable.

**10.** Composition anti-asthmatique contenant un composé selon une des revendications 1, 2 ou 3 ou un sel pharmaceutiquement acceptable de celui-ci.

**11.** Composition anti-allergique contenant un composé selon une des revendications 1, 2 ou 3 ou un sel pharmaceutiquement acceptable de celui-ci.

**12.** Composition pour prévenir ou traiter les rhinites allergiques contenant un composé selon une des revendications 1, 2 ou 3 ou un sel pharmaceutiquement acceptable de celui-ci.

**13.** Composition pour prévenir ou traiter les dermatites atopiques contenant un composé selon une des revendications

1, 2 ou 3 ou un sel pharmaceutiquement acceptable de celui-ci.

**14.** Composition selon la revendication 6 qui est sous forme de comprimés, de produit pour inhalation ou d'une fine poudre.

**15.** Composition selon la revendication 7 qui est sous forme de comprimés, de produit pour inhalation ou d'une fine poudre.

**16.** Composition selon la revendication 8 qui est sous forme de comprimés, de produit pour inhalation ou d'une fine poudre.

**17.** Composition selon la revendication 9 qui est sous forme de comprimés, de produit pour inhalation ou d'une fine poudre.

**18.** Composition selon la revendication 10 qui est sous forme de comprimés, de produit pour inhalation ou d'une fine poudre.

**19.** Composition selon la revendication 11 qui est sous forme de comprimés, de produit pour inhalation ou d'une fine poudre.

**20.** Composition selon la revendication 12 qui est sous forme de comprimés, de produit pour inhalation ou d'une fine poudre.

**21.** Composition selon la revendication 13 qui est sous forme de comprimés, de produit pour inhalation ou d'une fine poudre.

**22.** Utilisation d'un composé selon l'une des revendications 1, 2 ou 3, ou d'un sel pharmaceutiquement acceptable d'un tel composé pour la fabrication d'une composition anti-PAF

**23.** Utilisation d'un composé selon l'une des revendications 1, 2 ou 3, ou d'un sel pharmaceutiquement acceptable d'un tel composé pour la fabrication d'une composition pharmaceutique inhibitrice de la chimiotaxie des éosinophiles.

**24.** Utilisation d'un composé selon l'une des revendications 1, 2 ou 3, ou d'un sel pharmaceutiquement acceptable d'un tel composé pour la fabrication d'une composition anti-asthmatique.

**25.** Utilisation d'un composé selon l'une des revendications 1, 2 ou 3, ou d'un sel pharmaceutiquement acceptable d'un tel composé pour la fabrication d'une composition anti-allergique.

**26.** Utilisation d'un composé selon l'une des revendications 1, 2 ou 3, ou d'un sel pharmaceutiquement acceptable d'un tel composé pour la fabrication d'une composition pour la prévention ou le traitement des rhinites allergiques.

**27.** Utilisation d'un composé selon l'une des revendications 1, 2 ou 3, ou d'un sel pharmaceutiquement acceptable d'un tel composé pour la fabrication d'une composition pour la prévention ou le traitement des dermatites atopiques.